# EUROPÄISCHE PATENTSCHRIFT

(11) **EP 1 286 953 B1**
(45) Veröffentlichungstag und Bekanntmachung des Hinweises auf die Patenterteilung: **18.02.2004**
(21) Anmeldenummer: 01274059.3
(22) Anmeldetag: 19.10.2001
(51) Int. Cl.: C07C 237/20, A61K 7/13, D06P 1/32, C07D 295/18, C07D 295/12, C07D 231/38, C07D 307/52, C07D 211/46, C07D 207/27, C07D 213/75, C07D 233/61, C07D 307/22, C07D 317/66, C07D 207/08, C07D 207/16, C07D 211/42

(54) **(P-AMINO-HYDROXYPHENYL)-ACRYLAMID-DERIVATE UND DIESE VERBINDUNGEN ENTHALTENDE FÄRBEMITTEL**
(P-AMINO-HYDROXYPHENYL)-ACRYLAMIDE DERIVATIVES AND DYES CONTAINING SAID COMPOUNDS
DERIVES (P-AMINO-HYDROXYPHENYL)-ACRYLAMIDE ET COLORANTS CONTENANT CES COMPOSES

(30) Priorität: 30.03.2001 DE 10115994
(43) Veröffentlichungstag der Anmeldung: 05.03.2003
(73) Patentinhaber: Wella Aktiengesellschaft, 64274 Darmstadt (DE)
(72) Erfinder: CHASSOT, Laurent, 1724 Praroman (CH); BRAUN, Hans-Jürgen, 3182 Überstorf (CH)
(86) Internationale Anmeldenummer: PCT/EP2001/012126
(87) Internationale Veröffentlichungsnummer: WO 2002/079144

(56) Entgegenhaltungen:
- DE-A- 19 607 751

## Beschreibung

Die Erfindung betrifft neue (p-Amino-hydroxyphenyl)-acrylamid-Derivate sowie diese Verbindungen enthaltende Mittel zum Färben von Keratinfasern, insbesondere menschlichen Haaren.

Auf dem Gebiet der Färbung von Keratinfasem, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kuppiersubstanzen in Gegenwart eines geeigneten Oxidationsmittels. Als Entwicklersubstanzen werden hierbei insbesondere 2,5-Diaminotoluol, 2,5-Diaminophenylethylalkohol, p-Aminophenol, 1,4-Diaminobenzol und 4,5-Diaminopyrazol-1-(2-hydroxyethyl) eingesetzt, während als Kupplersubstanzen beispielsweise Resorcin, 2-Methyl-resorcin, 1-Naphthol, 3-Aminophenol, m-Phenylendiamin, 2-Amino-4-(2'-hydroxyethyl)amino-anisol, 1,3-Diamino-4-(2'hydroxyethoxy)benzol und 2,4-Diamino-5-fluor-toluol zu nennen sind.

An Oxidationsfarbstoffe, die zur Färbung menschlicher Haare verwendet werden, werden neben der Färbung in der gewünschten Intensität zahlreiche zusätzliche Anforderungen gestellt. So müssen die Farbstoffe in toxikologischer und dermatotogischer Hinsicht unbedenklich sein und die erzielten Haarfärbungen eine gute Lichtechtheit, Dauerwellechtheit, Säureechtheit und Reibeechtheit aufweisen. Auf jeden Fall aber müssen solche Färbungen ohne Einwirkung von Licht, Reibung und chemischen Mitteln über einen Zeitraum von mindestens 4 bis 6 Wochen stabil bleiben. Außerdem ist es erforderlich, dass durch Kombination geeigneter Entwickiersubstanzen und Kupplersubstanzen eine breite Palette verschiedener Farbnuancen erzeugt werden kann.

Zur Erzielung nartürlicher und besonders modischer Nuancen im Rotbereich wird vor allem p-Aminophenol, allein oder im Gemisch mit anderen Entwicklersubstanzen, in Kombination mit geeigneten Kuppiersubstanzen eingesetzt. Es wurde bereits versucht, die Eigenschaften von p-Aminophenolen durch die Einführung von Substituenten zu verbessern. In diesem Zusammenhang sei auf die DE-OS 196 07 751 verwiesen, in der Färbemittel beschrieben werden, welche als Entwicklersubstanzen spezielle, substituierte p-Aminophenol-Derivate, beispielsweise 5-Amino-2-hydroxyzimtsäure, enthalten.

Mit den derzeit bekannten Färbemitteln ist es jedoch nicht möglich, die an ein Färbemittel gestellten Anforderungen in allen Punkten zu erfüllen. Es bestand daher weiterhin ein Bedürfnis nach neuen Entwicklersubstanzen, welche die vorgenannten Anforderung in besonderem Masse erfüllen.

Hierzu wurde nun überraschenderweise gefunden, daß neue (p-Amino-hydroxyphenyl)-acrylamid-Derivate gemäß der allgemeinen Formel (I) die an Entwicklersubstanzen gestellten Anforderungen in besonders hohem Masse erfüllen. So werden bei Verwendung dieser Entwicklersubstanzen mit den meisten bekannten Kupplersubstanzen farbstarke Farbnuancen erhalten, die außerordentlich lichtecht und waschecht sind.

Gegenstand der vorliegende Erfindung sind daher (p-Amino-hydroxyphenyl)-acrylamid-Derivate der allgemeinen Formel (I) oder deren physiologisch verträgliche, wasserlösliche Salze, 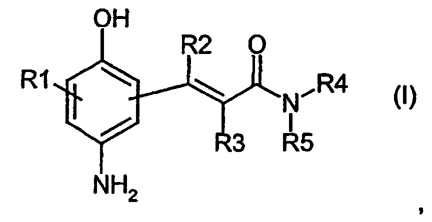 worin
**R1** gleich Wasserstoff, einem Halogenatom (F, Cl, Br, J), einer C₁-C₄-Alkylgruppe, einer C₁-C₄-Hydroxyalkylgruppe oder einer C₁-C₄-Alkoxygruppe ist;
**R2** und **R3** unabhängig voneinander gleich Wasserstoff oder einer C₁-C₆-Alkylgruppe sind;
**R4** und **R5** unabhänging voneinander gleich Wasserstoff, einer C₁-C₂-Alkoxygruppe, einer C₁-C₆-Alkylgruppe, einer ungesättigten C₃-C₆-Alkylgruppe, einer C₂-C₄-Hydroxyalkylgruppe, einer C₃-C₄-Dihydroxyalkylgruppe, einer C₂-C₄-Aminoalkylgruppe, einer C₂-C₄-Dimethylaminoalkylgruppe, einer C₂-C₄-Acetylaminoalkylgruppe, einer C₂-C₄-Methoxyalkylgruppe, einer C₂-C₄-Ethoxyalkylgruppe, einer C₁-C₄-Cyanoalkylgruppe, einer C₁-C₄-Carboxyalkylgruppe, einer C₁-C₄-Aminocarbonylalkylgruppe, einer Pyridylmethylgruppe, einer Furfurylgruppe, einer Thienylmethylgruppe, einer hydrierten Furfurylgruppe, einer substituierten Pyridylgruppe, einem Rest der Formel (II) 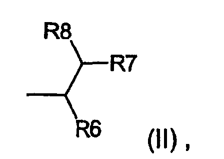 einem Rest der Formel (III) 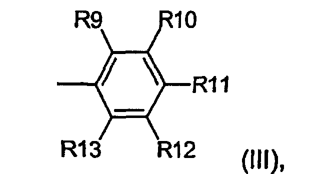 oder einem Rest der Formel (IV) 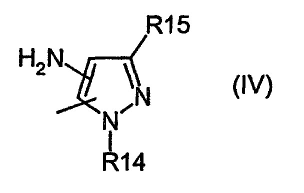 sind, oder R4 und R5 gemeinsam mit dem Stickstoffatom einen Ring der Formel 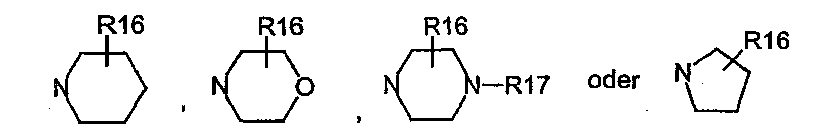 bilden;
**R6** gleich Wasserstoff, einer Carboxygruppe oder einer Aminocarbonylgruppe ist;
**R7** und **R8** unabhänging voneinander gleich Wasserstoff, einer Hydroxygruppe, einer Aminocarbonylgruppe, einer Methylthiomethylgruppe, einem mit einer Phenylgruppe oder einer Hydroxygruppe substituierten Phenylrest oder einem Rest der Formel 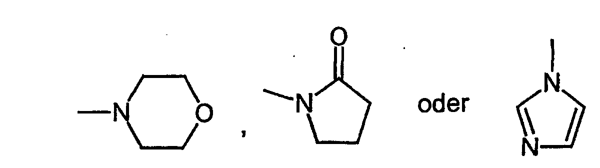 sind
**R9, R10, R11, R12** und **R13** unabhängig voneinander Wasserstoff, ein Halogenatom (F, Cl, Br, J), eine Cyanogruppe, eine Hydroxygruppe, eine C₁-C₄-Alkoxygruppe, eine C₁-C₄-Hydroxyalkoxygruppe, eine C₁-C₆-Alkylgruppe, eine C₁-C₄-Alkylthioethergruppe, eine Mercaptogruppe, eine Nitrogruppe, eine Aminogruppe, eine Alkylaminogruppe, eine C₁-C₄-Hydroxyalkylaminogruppe, eine Dialkylaminogruppe, eine Di(C₁-C₄hydroxyalkyl)aminogruppe, eine (C₃-C₄-Dihydroxyalkyl)aminogruppe, eine (C₁-C₄-Hydroxyalkyl)- C₁-C₄-alkylaminogruppe, eine Trifluormethan-gruppe, eine -C(O)H-Gruppe, eine -C(O)CH₃-Gruppe, eine -C(O)CF₃-Gruppe, eine -Si(CH₃)₃-Gruppe, eine C₁-C₄-Hydroxyalkylgruppe oder eine C₃-C₄ Dihydroxyalkylgruppe darstellen, oder zwei nebeneinanderliegende Reste R9 bis R13 eine -O-CH2-O-Brücke bilden;
**R14** gleich einer C₁-C₄-Alkylgruppe einer Benzylgruppe oder einer C₂-C₄-Hydroxyalkylgruppe ist;
**R15** gleich Wasserstoff oder einer C₁-C₆ Alkylgruppe ist;
**R16** gleich Wasserstoff, einer Hydroxygruppe, einer Carboxygruppe, einer Aminocarbonylgruppe oder einer Hydroxymethylgruppe ist; und
**R17** gleich Wasserstoff oder einer C₁-C₆-Alkylgruppe ist.

Als geeignete Verbindungen der Formel (I) können beispielweise die folgenden Verbindungen genannt werden: 3-(5-Amino-2-hydroxyphenyl)-N-cyclopropyl-acrylamid, 3-(5-Amino-2-hydroxyphenyl)-N-propyl-acrylamid, 3-(5-Amino-2-hydroxyphenyl)-1-pyrrolidin-1-yl-propenon, 3-(5-Amino-2-hydroxyphenyl)-N-(2-methoxyethyl)-acrylamid, 3-(5-Amino-2-hydroxyphenyl)-1-morpholin-4-yl-propenon, 3-(5-Amino-2-hydroxyphenyl)-N-(1-hydroxymethylpropyl)-acrylamid, 3-(5-Amino-2-hydroxyphenyl)-N-furan-2-ylmethyl-acrylamid, 3-(5-Amino-2-hydroxyphenyl)-N-methoxy-N-methylacrylamid, 3-(5-Amino-2-hydroxyphenyl)-1-(4-methyl-piperazin-1-yl)-propenon, 3-(5-Amino-2-hydroxyphenyl)-1-(4-hydroxy-piperidin-1-yl)-propenon, N-(2-Acetylaminoethyl)-3-(5-amino-2-hydroxyphenyl)-acrylamid, 3-(5-Amino-2-hydroxyphenyl)-N-(2-morpholin-4-yl-ethyl)-acrylamid, 3-(5-Amino-2-hydroxyphenyl)-N-[3-(2-oxo-pyrrolidin-1-yl)-propyl]-acrylamid, N-Allyl-3-(5-amino-2-hydroxyphenyl)-acrylamid, 3-(5-Amino-2-hydroxyphenyl)-N-(2-hydroxy-1-methylethyl)-acrylamid, 3-(5-Amino-2-hydroxyphenyl)-N-[2-(5-nitro-pyridin-2-ylamino)-ethyl]-acrylamid, N-(2-Aminoethyl)-3-(5-amino-2-hydroxyphenyl)-acrylamid, 3-(5-Amino-2-hydroxyphenyl)-N-(3-imidazol-1-yl-propyl)-acrylamid, 3-(5-Amino-2-hydroxyphenyl)-N-(tetrahydrofuran-2-ylmethyl)-acrylamid, 3-(5-Amino-2-hydroxyphenyl)-N-(4-aminophenyl)-acrylamid, N-[4-Amino-2(3)-(2-hydroxyethyl)-phenyl]-3-(5-amino-2-hydroxyphenyl)-acrylamid, 3-(5-Amino-2-hydroxyphenyl)-N-{4-[bis-(2-hydroxyethyl)-amino]-phenyl}-acrylamid, 3-(5-Amino-2-hydroxyphenyl)-N-(3-aminophenyl)-acrylamid, N-[5-Amino-2(4)-(2-hydroxyethoxy)-phenyl]-3-(5-amino-2-hydroxyphenyl)-acrylamid, 3-(5-Amino-2-hydroxyphenyl)-N-[2-chloro-4-(2-hydroxyethylamino)-5-nitro-phenyl]-acrylamid, N-[4-Amino-2-(2-hydroxyethyl)-2H-pyrazol-3-yl]-3-(5-amino-2-hydroxyphenyl)-acrylamid, 3-(5-Amino-2-hydroxyphenyl)-N-benzo[1,3]dioxol-5-yl-acrylamid, 3-(5-Amino-2-hydroxyphenyl)-N-(2-hydroxyethyl)-N-methyl-acrylamid, 3-(5-Amino-2-hydroxyphenyl)-N-ethyl-N-(2-hydroxyethyl)-acrylamid, 3-(5-Amino-2-hydroxyphenyl)-1-(2-hydroxymethyl-pyrrolidin-1-yl)-propenon, 1-[3-(5-Amino-2-hydroxyphenyl)-acryloyl]-pyrrolidin-2-carbonsäureamid, 3-(5-Amino-2-hydroxyphenyl)-1-(3-hydroxy-piperidin-1-yl)-propenon, 3-(5-Amino-2-hydroxyphenyl)-N-(2-hydroxy-1 -hydroxymethylethyl)-acrylamid, 3-(5-Amino-2-hydroxyphenyl)-N-ethyl-acrylamid, 2-[3-(5-Amino-2-hydroxyphenyl)-acryloylamino]-3-methyl-buttersäure, 3-(5-Amino-2-hydroxyphenyl)-N-(4-hydroxyphenyl)-acrylamid, 3-(5-Amino-2-hydroxyphenyl)-N-(1-carbamoyl-2-hydroxyethyl)-acrylamid, 3-(5-Amino-2-hydroxyphenyl)-N-(4-amino-2(3)-methyl-phenyl)-acrylamid, 3-(5-Amino-2-hydroxyphenyl)-N-(3-hydroxy-4-methylphenyl)-acrylamid, 3-(5-Amino-2-hydroxyphenyl)-N-(2-hydroxy-5-nitro-phenyl)-acrylamid, 3-(5-Amino-2-hydroxyphenyl)-acrylsäure, 3-(2-Amino-5-hydroxyphenyl)-N-cyclopropylacrylamid, 3-(2-Amino-5-hydroxyphenyl)-N-propyl-acrylamid, 3-(2-Amino-5-hydroxyphenyl)-1-pyrrolidin-1-yl-propenon, 3-(2-Amino-5-hydroxyphenyl)-N-(2-methoxyethyl)-acrylamid, 3-(2-Amino-5-hydroxyphenyl)-1-morpholin-4-ylpropenon, 3-(2-Amino-5-hydroxyphenyl)-N-(1-hydroxymethylpropyl)-acrylamid, 3-(2-Amino-5-hydroxyphenyl)-N-furan-2-ylmethyl-acrylamid, 3-(2-Amino-5-hydroxyphenyl)-N-methoxy-N-methyl-acrylamid, 3-(2-Amino-5-hydroxyphenyl)-1-(4-methyl-piperazin-1-yl)-propenon, 3-(2-Amino-5-hydroxyphenyl)-1-(4-hydroxy-piperidin-1-yl)-propenon, N-(2-Acetylaminoethyl)-3-(2-Amino-5-hydroxyphenyl)-acrylamid, 3-(2-Amino-5-hydroxyphenyl)-N-(2-morpholin-4-yl-ethyl)-acrylamid, 3-(2-Amino-5-hydroxyphenyl)-N-[3-(2-oxo-pyrrolidin-1-yl)-propyl]-acrylamid, N-Allyl-3-(2-Amino-5-hydroxyphenyl)-acrylamid, 3-(2-Amino-5-hydroxyphenyl)-N-(2-hydroxy-1-methytethyl)-acrylamid, 3-(2-Amino-5-hydroxyphenyl)-N-[2-(5-nitro-pyridin-2-ylamino)-ethyl]-acrylamid, N-(2-Aminoethyl)-3-(2-Amino-5-hydroxyphenyl)-acrylamid, 3-(2-Amino-5-hydroxyphenyl)-N-(3-imidazol-1-yl-propyl)-acrylamid, 3-(2-Amino-5-hydroxyphenyl)-N-(tetrahydrofuran-2-ylmethyl)-acrylamid, 3-(2-Amino-5-hydroxyphenyl)-N-(4-aminophenyl)-acrylamid, N-[4-Arnino-2(3)-(2-hydroxyethyl)-phenyl]-3-(2-Amino-5-hydroxyphenyl)-acrylamid, 3-(2-Amino-5-hydroxyphenyl)-N-{4-[bis-(2-hydroxyethyl)-amino]-phenyl)-acrylamid, 3-(2-Amino-5-hydroxyphenyl)-N-(3-aminophenyl)-acrylamid, N-[5-Amino-2 (4)-(2-hydroxyethoxy)-phenyl]-3-(2-Amino-5-hydroxyphenyl)-acrylamid, 3-(2-Amino-5-hydroxyphenyl)-N-[2-chloro-4-(2-hydroxyethylamino)-5-nitro-phenyl]-acrylamid, N-[4-Amino-2-(2-hydroxyethyl)-2H-pyrazol-3-yl]-3-(2-Amino-5-hydroxyphenyl)-acrylamid, 3-(2-Amino-5-hydroxyphenyl)-N-benzo[1,3]dioxol-5-yl-acrylamid, 3-(2-Amino-5-hydroxyphenyl)-N-(2-hydroxyethyl)-N-methyl-acrylamid, 3-(2-Amino-5-hydroxyphenyl)-N-ethyl-N-(2-hydroxyethyl)-acrylamid, 3-(2-Amino-5-hydroxyphenyl)-1-(2-hydroxymethyl-pyrrolidin-1-yl)propenon, 1-[3-(2-Amino-5-hydroxyphenyl)-acryloyl]-pyrrolidin-2-carbonsäureamid, 3-(2-Amino-5-hydroxyphenyl)-1-(3-hydroxy-piperidin-1-yl)-propenon, 3-(2-Amino-5-hydroxyphenyl)-N-(2-hydroxy-1-hydroxymethylethyl)-acrylamid, 3-(2-Amino-5-hydroxyphenyl)-N-ethyl-acrylamid, 2-[3-(2-Amino-5-hydroxyphenyl)-acryloylamino]-3-methyl-buttersäure, 3-(2-Amino-5-hydroxyphenyl)-N-(4-hydroxyphenyl)-acrylamid, 3-(2-Amino-5-hydroxyphenyl)-N-(1-carbamoyl-2-hydroxyethyl)-acrylamid, 3-(2-Amino-5-hydroxyphenyl)-N-(4-amino-2(3)-methylphenyl)-acrylamid, 3-(2-Amino-5-hydroxyphenyl)-N-(3-hydroxy-4-methylphenyl)-acrylamid, 3-(2-Amino-5-hydroxyphenyl)-N-(2-hydroxy-5-nitro-phenyl)-acrylamid und 3-(2-Amino-5-hydroxyphenyl)-acrylsäure sowie deren physiologisch verträgliche Salze.

Bevorzugt sind Verbindungen der Formel (I), bei denen (i) eine, mehrere oder alle Restgruppen **R1, R2** und **R3** gleich Wasserstoff sind und/oder (ii) **R4** gleich einer C₁-C₂-Alkylgruppe, einer Methoxygruppe oder einer C₂-C₄-Hydroxyalkylgruppe ist und **R5** gleich einer C₂-C₄-Hydroxyalkylgruppe ist und/oder (iii) **R4** und **R5** unabhängig voneinander gleich Wasserstoff, einer C₁-C₄-Alkylgruppe, einer ungesättigen C₁-C₆-Alkylgruppe einer C₂-C₄-Hydroxyalkylgruppe, einer C₃-C₄-Dihydroxyalkylgruppe, einem Furfurylrest, einem substituierten Phenylrest der Formel (III) oder einem substituierten Pyrazolylrest der Formel (IV) sind und/oder (iv) **R4** gleich Wasserstoff und **R5** gleich einer C₁-C₄- Alkylgruppe, einer ungesättigten C₃-C₆-Alkylgruppe, einer C₂-C₄-Hydroxyalkylgruppe, einer C₃-C₄-Dihydroxyalkylgruppe, einem Furfurylrest, einem substituierten Phenylrest der Formel (III) oder einem substituierten Pyrazolylrest der Formel (IV) ist.

Besonders bevorzugt sind die folgenden Verbindungen der Formel (I): 3-(5-Amino-2-hydroxyphenyl)-N-ethyl-acrylamid, 3-(2-Amino-5-hydroxyphenyl)-N-ethyl-acrylamid, 3-(5-Amino-2-hydroxyphenyl)-N-(4-hydroxyphenyl)-acrylamid, 3-(2-Amino-5-hydroxyphenyl)-N-(4-hydroxyphenyl)-acrylamid, 3-(5-Amino-2-hydroxyphenyl)-N-(4-aminophenyl)-acrylamid, 3-(2-Amino-5-hydroxyphenyl)-N-(4-aminophenyl)-acrylamid, N-[4-Amino-2-(2-hydroxyethyl)-2H-pyrazol-3-yl]-3-(5-amino-2-hydroxyphenyl)-acrylamid und N-[4-Amino-2-(2-hydroxyethyl)-2H-pyrazol-3-yl]-3-(2-amino-5-hydroxyphenyl)-acrylamid sowie deren physiologisch verträgliche Salze.

Die Verbindungen der Formel (I) können sowohl als freie Basen als auch in Form ihrer physiologisch verträglichen Salze mit anorganischen oder organischen Säuren, wie zum Beispiel Salzsäure, Schwefelsäure, Phosphorsäure, Essigsäure, Propionsäure, Milchsäure oder Zitronensäure, eingesetzt werden.

Die Herstellung der erfindungsgemäßen Aminophenol-Derivate der Formel (I) kann unter Verwendung von literaturbekannten Syntheseverfahren erfolgen. Die Synthese der erfindungsgemäßen Verbindungen kann beispielsweise wie folgt durchgeführt werden: Durch eine Aminolyse eines substituierten Benzols der Formel (V) 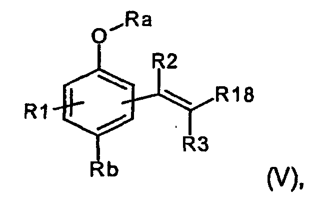 mit einem Amin der Formel NHR4R5 und anschliessende Abspaltung der Schutzgruppe und/oder Reduktion der Nitrogruppe,
worin **Ra** für eine geeignete Schutzgruppe, wie sie zum Beispiel in Organic Synthesis, Kapitel 3, "Protection for Phenols", Seite 143 ff., Wiley Interscience, 1991 beschrieben wird, steht; **Rb** die Bedeutung NHRa oder NO₂ hat, **R18** eine Carbonsäure-Gruppe, eine Carbonsäurechlorid-Gruppe, eine Carbonsäureester-Gruppe oder eine Carbonsäureanhydrid-Gruppe darstellt, und die Reste **R1, R2, R3, R4** und **R5** die in Formel (I) angegebene Bedeutung haben.

Die erfindungsgemäßen Verbindungen der Formel (I) sind insbesondere als Entwicklersubstanzen in Oxidationsfärbemitteln für Keratinfasern verwendbar und ermöglichen eine breite Palette verschiedener Farbnuancen, welche sich von blonden über braune, purpurne bis hin zu violetten Farbtönen erstrecken.

Ein weiterer Gegenstand der vorliegenden Erfindung sind daher Mittel zum oxidativen Färben von Keratinfasern, wie zum Beispiel Haaren, Pelzen, Federn oder Wolle, insbesondere menschlichen Haaren, auf der Basis einer Entwicklersubstanz-Kupplersubstanz-Kombination, welche als Entwicklersubstanz mindestens ein (p-Amino-hydroxyphenyl)-acrylamid-Derivat der Formel (I) enthalten.

Das Aminophenol-Derivat der Formel (I) ist in dem erfindungsgemäßen Färbemittel in einer Menge von 0,005 bis 20 Gewichtsprozent enthalten, wobei eine Menge von 0,01 bis 5,0 Gewichtsprozent und insbesondere 0,1 bis 2,5 Gewichtsprozent bevorzugt ist.

Als Kupplersubstanzen kommen vorzugsweise N-(3-Dimethylaminophenyl)-harnstoff, 2,6-Diamino-pyridin, 2-Amino-4-[(2-hydroxyethyl)-amino]-anisol, 2,4-Diamino-1-fluor-5-methyl-benzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxy-ethyl)amino]-1,5-dimethoxy-benzol, 2,3-Diamino-6-methoxy-pyridin, 3-Amino-6-methoxy-2-(methytamino)-pyridin, 2,6-Diamino-3,5-dimethoxypyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 1,3-Diamino-4-(3-hydroxypropoxy)-benzol, 1,3-Diamino-4-(2-methoxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methy(amino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-4-methoxy-2-methyl-phenol, 5-[(2-Hydroxyethyl)amino]-2-methyl-phenol,3-[(2-Hydroxyethyl)amino]-phenol, 3-[(2-Methoxyethyl)amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methyl-phenol, 2-Amino-3-hydroxy-pyridin, 2,6-Dihydroxy-3,4-dimethylpyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion in Betracht.

Obwohl die vorteilhaften Eigenschaften der hier beschriebenen Aminophenol-Derivate der Formel (I) es nahelegen, diese als alleinige Entwicklersubstanz zu verwenden, ist es selbstverständlich auch möglich, die p-Aminophenol-Derivate der Formel (I) gemeinsam mit bekannten Entwicklersubstanzen, wie zum Beispiel 1,4-Diaminobenzol, 2,5-Diaminotoluol, 2-(2,5-Diaminophenyl)-ethylalkohol, 1-(2,5-Diaminophenyl)-ethylalkohol, N,N-Bis-(2'-hydroxyethyl)-1,4-diaminobenzol, 4-Aminophenol und dessen Derivaten, wie zum Beispiel 4-Amino-3-methylphenol, Pyrazolderivaten, wie zum Beispiel 4,5-Diamino-1-(2-hydroxyethyl)-pyrazol, 4,5-Diamino-1-benzyl-pyrazof und 4,5-Diamino-1-(4-methylbenzyl)-pyrazol, oder Tetraaminopyrimidinen, einzusetzen.

Die Kupplersubstanzen und Entwicklersubstanzen können in dem erfindungsgemäßen Färbemittel jeweils einzeln oder im Gemisch miteinander enthalten sein, wobei die Gesamtmenge an Kupplersubstanzen und Entwicklersubstanzen in dem erfindungsgemäßen Färbemittel (bezogen auf die Gesamtmenge des Färbemittels) jeweils 0,005 bis 20 Gewichtsprozent, vorzugsweise 0,01 bis 5 Gewichtsprozent und insbesondere 0,1 bis 2,5 Gewichtsprozent, beträgt.

Die Gesamtmenge der in dem hier beschriebenen Färbemittel enthaltenen Entwicklersubstanz-Kupplersubstanz-Kombination beträgt vorzugsweise 0,01 bis 20 Gewichtsprozent, wobei eine Menge von 0,02 bis 10 Gewichtsprozent und insbesondere 0,2 bis 6,0 Gewichtsprozent besonders bevorzugt ist. Die Entwicklersubstanzen und Kupplersubstanzen werden im allgemeinen in etwa äquimolaren Mengen eingesetzt; es ist jedoch nicht nachteilig, wenn die Entwicklersubstanzen diesbezüglich in einem gewissen Überschuß oder Unterschuß vorhanden sind.

Weiterhin kann das erfindungsgemäße Färbemittel zusätzlich andere Farbkomponenten, beispielsweise 6-Amino-2-methytphenol und 2-Amino-5-methylphenol, sowie ferner übliche direktziehende Farbstoffe, zum Beispiel Triphenylmethanfarbstoffe wie 4-[(4'-aminophenyl)-(4'-imino-2",5"cyclohexadien-1"-yliden)methyl]-2-methylaminobenzol-monohydrochlorid (C.I. 42 510) und 4-[(4'amino-3'-methyl-phenyl)-(4"-imino-3"-methyl-2",5"cyclohexadien-1"-yliden)methyl]-2-methyl-aminobenzol monohydrochlorid (C.I. 42 520), aromatische Nitrofarbstoffe wie 4-(2'-hydroxyethyl)amino-nitrotoluol, 2-Amino-4,6-dinitrophenol, 2-Amino-5-(2'-hydroxyethyl)amino-nitrobenzol, 2-Chlor-6-(ethylamino)-4-nitrophenol, 4-Chlor-N-(2-hydroxyethyl)-2-nitroanilin, 5-Chlor-2-hydroxy-4-nitroanilin, 2-Amino-4-chlor-6-nitrophenol und 1-[(2'-Ureidoethyl)amino-4-nitrobenzol, Azofarbstoffe wie 6-[(4'-Aminophenyl)azo]-5hydroxynaphthalin-1-sulfonsäure-Natriumsalz (C.l. 14 805) und Dispersionsfarbstoffe wie beispielsweise 1,4-Diaminoanthrachinon und 1,4,5,8-Tetraaminoantrachinon, enthalten. Die Färbemittel können diese Farbkomponenten in einer Menge von 0,1 bis 4 Gewichtsprozent enthalten.

Selbstverständlich können die Kupplersubstanzen und Entwicklersubstanzen sowie die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Darüber hinaus können in den Färbemitteln, falls diese zur Färbung von Haaren verwendet werden sollen, noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein.

Die Zubereitungsform des erfindungsgemäßen Färbemittels kann beispielsweise eine Lösung, insbesondere eine wässrige oder wässrigalkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie höhere Fettalkohole, Stärke, Cellulosederivate, Petrolatum, Paraffinöl und Fettsäuren, sowie außerdern Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von 0,5 bis 30 Gewichtsprozent, die Verdicker in einer Menge von 0,1 bis 30 Gewichtsprozent und die Pflegestoffe in einer Konzentration von 0.1 bis 5 Gewichtsprozent.

Je nach Zusammensetzung kann das erfindungsgemäße Färbemittel schwach sauer, neutral oder alkalisch reagieren. Insbesondere weist es einen pH-Wert von 6,5 bis 11,5 auf, wobei die basische Einstellung vorzugsweise mit Ammoniak erfolgt. Es können aber auch organische Amine, zum Beispiel Monoethanolamin und Triethanolamin, oder auch anorganische Basen wie Natriumhydroxid und Kaliumhydroxid Verwendung finden. Für eine pH-Einstellung im sauren Bereich kommen anorganische oder organische Säuren, zum Beispiel Phosphorsäure, Essigsäure Zitronensäure oder Weinsäure, in Betracht.

Für die Anwendung zur oxidativen Färbung von Haaren vermischt man das vorstehend beschriebene Färbemittel unmittelbar vor dem Gebrauch mit einem Oxidationsmittel und trägt eine für die Haarfärbebehandlung ausreichende Menge, je nach Haarfülle, im allgemeinen 60 bis 200 Gramm, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin, Natriumborat oder Natriumcarbonat in Form einer 3-bis 12prozentigen, vorzugsweise 6prozentigen, wässrigen Lösung, aber auch Luftsauerstoff in Betracht. Wird eine 6prozentige Wasserstoff-peroxid-Lösung als Oxidationsmittel verwendet, so beträgt das Gewichtsverhältnis zwischen Haarfärbemittel und Oxidationsmittel 5:1 bis 1:2, vorzugeweise jedoch 1:1. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Man läßt das Gemisch bei 15 bis 50 Grad Celsius 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Die erfindungsgemäßen Haarfärbemittel mit einem Gehalt an p-Aminophenol-Derivaten der Formel (I) als Entwicklersubstanz ermöglichen Haarfärbungen mit ausgezeichneter Farbechtheit, insbesondere was die Lichtechtheit, Waschechtheit und Reibeechtheit anbetrifft. Hinsichtlich der färberischen Eigenschaften bieten die erfindungsgemäßen Haarfärbemittel je nach Art und Zusammensetzung der Farbkomponenten eine breite Palette verschiedener Farbnuancen, welche sich von blonden Ober braune, purpume, violette bis hin zu blauen und schwarzen Farbtönen erstreckt. Hierbei zeichnen sich die Farbtöne durch ihre besondere Farbintensität und einen guten Farbausgleich zwischen geschädigtem und ungeschädigtem Haar aus. Die sehr guten färberischen Eigenschaften der Haarfärbemittel gemäß der vorliegenden Anmeldung zeigen sich weiterhin darin, dass diese Mittel eine Anfärbung von ergrauten, chemisch nicht vorgeschädigten Haaren problemlos und mit guter Deckkraft ermöglichen.

Die nachfolgenden Beispiele sollen den Gegenstand der Erfindung näher erläutern.

### Beispiele

### Beispiel 1: Synthese von (p-Amino-hydroxyphenyl)-acrylamid-Derivaten der Formel (I) (Allgemeine Synthesevorschrift)

### A. Synthese von N-(3-Brom-4-hydroxyphenyl)-carbaminsäure-tert.butylester

Zu einer Suspension von 10 g (47,8 mmol) N-(4-Hydroxyphenyl)-carbaminsäure-tert.butylester in 100 ml Chloroform tropft man innerhalb von 2 Stunden bei 0 °C eine Lösung von 9,4 g (52,8 mmol) N-Bromsuccinimid in 450 ml Chloroform. Die Reaktionsmischung wird anschließend weitere 15 Minuten lang gerührt, sodann zweimal mit Wasser (zuerst 400 ml, dann 200 ml) gewaschen, mit Magnesiumsulfat getrocknet, filtriert und teilweise eingeengt. Der Rückstand wird sodann unter Rühren mit Hexan versetzt, wobei sich ein Niederschlag bildet. Der Niederschlag wird abfiltriert und mit Hexan gewaschen.
Es werden 9,7 g (70 % der Theorie) N-(3-Brom-4-hydroxyphenyl)-carbaminsäure-tert.butylester erhalten.

### B. Synthese von N-(3-Brom-4-ethoxymethoxy-phenyl)-carbaminsäure-tert.butylester

Zu einer Lösung von 5 g (17,4 mmol) N-(3-Brom-4-hydroxyphenyl)-carbaminsäure-tert.butylester in 60 ml Teratohydrofuran werden bei 0°C portionsweise 0,76 g (17,4 mmol) einer Natriumhydrid-Dispersion (55% in Öl) gegeben. Das Gemisch wird anschließend 50 Minuten lang bei 0 °C gerührt und sodann mit 1,83g (19,4 mmol) Chlormethylethylether versetzt. Das Gemisch wird eine weiter Stunde lang bei 0°C gerührt und sodann auf Eis gegossen, mit Essigsäureethylester extrahiert und die organische Phase mit einer gesättigten wässrigen Kochsalz-Lösung gewaschen, über Na₂SO₄ getrocknet und nach Filtration eingeengt.
Der Rückstand wird an Kieselgel mit Petrolether/Essigsäureethylester (9:1) gereinigt.
Es werden 4,8 g (80 % der Theorie) N-(3-Brom-4-ethoxymethoxy-phenyl)-carbaminsäure-tert.butylester erhalten.
¹H-NMR (300 MHz, CDCl₃): δ = 7,67 (d, 1H); 7,16 (dd, 1H); 7,07 (d, 1H); 7,21 6,40 (br, 1H); 5,23 (s, 2H); 3,77 (q, 2H); 1,51 (s, 9H); 1,22 (t, 3H)

### C. Synthese von N-(4-ethoxymethoxy -3-formylphenyl)-carbaminsäure-tert.butylester

3,3 g (0,01 mol) (3-Brom-4-tert-butoxycarbonylamino-phenyl)-carbaminsäure tert.butylester aus Stufe **A** werden unter Argon in 100 ml wasserfreiem Tetrahydrofuran gelöst. Schrittweise werden sodann 17 ml (= 0,03 mol) einer 1,6 molaren etherischen Methyllithiumlösung zugegeben. Die Reaktionsmischung wird auf -20 °C abgekühlt, und schrittweise mit 7 ml (= 0,01 mol) einer 1,5 molaren tert.-Butyllithiumlösung versetzt. Nach beendeter Zugabe wird die Lösung noch 30 Minuten lang bei der angegebenen Temperatur gerührt. Anschließend werden 1,2 g (0,02 mol) Dimethylformamid zugegeben und die Reaktionmischung wird sodann eine Stunde lang bei -20 °C gerührt. Nach langsamer Erwämung auf 0 °C wird die Reaktionsmischung mit einer 10%igen Phosphatpufferlösung hydrolisiert und dann auf Essigsäureethylester gegossen. Die wässerige Phase wird sodann mit Essigsäureethylester extrahiert und die organische Phase mit Magnesiumsulfat getrocknet. Das Lösungsmittel wird am Rotationsverdampfer abdestilliert und der Rückstand mit Hexan versetzt. Der erhaltene gelbe Niederschlag wird anschließend abfiltriert, mit Hexan gewaschen und getrocknet
Es werden 2,0 g (70 % der Theorie) N-(4-ethoxymethoxy-3-formylphenyl)-carbaminsäure-tert.butylester erhalten.
¹H-NMR (300 MHz, CDCl₃): δ = 10,43 (s,1H); 7,73 (d, 1H); 7,61 (d, 1H); 7,20 (d, 1H); 6,45 (br, 1H); 5,31 (s, 2H); 3,75 (q, 2H); 1,51 (s, 9H); 1,23 (t, 3H)

### D. Synthese von 3-(5-tert-butoxycarbonylamino-2-ethoxymethoxyphenyl)-acrylsäuremethylester

8,9 g (0,03 mol) von N-(4-ethoxymethoxy-2-formylphenyl)-carbaminsäure-tert.butylester aus Stufe **C** werden in 70 ml Tetrahydrofuran gelöst und mit 11,9 g (0,036 mol) Methoxycarbonyl-methylen-triphenylphosphoran versetzt. Die Reaktionmsichung wird 3 Stunden lang bei Raumtemperatur gerührt. Anschließend wird das Reaktionsgemisch auf Wasser gegossen, mit Essigsäureethylester extrahiert und die organische Phase mit einer gesättigten wässrigen Kochsalzlösung gewaschen, über Natriumsulfat getrocknet und nach Filtration eingeengt.
Die Flash-Chromatographie des Rohproduktes an Kieselgel mit Hexan/Essigsäureethylester ergibt 10,5 g (95 % der Theorie).
Das reine E-Isomer wird durch Suspendieren des Gemisches in Hexan/Diethylether (10:1) und nachfolgende Umkristallisation aus Essigsäureethylester/Hexan gewonnen.
¹H-NMR (300 MHz, CDCl₃ δ = 7,99 (d, 1H); 7,60 (br s, 1H); 7,26 (dd, 1H); 7,12 (d, 1H); 6,50 (d, 1H); 6,42 (br s, 1H); 5,25 (s, 2H); 3,79 (s, -3H); 3,73 (q, 2H); 1,51 (s, 9H); 1,21 (t, 3H)

### E. Synthese von 3-(5-tert-butoxycarbonylamino-2-ethoxymethoxyphenyl)-acrylsäure

Zu einer Lösung von 6,3 g (0,018 mol) des 3-(5-tert-butoxycarbonylamino-2-ethoxymethoxy-phenyl)-acrylsäuremethylesters aus Stufe **D** in 50 ml Tetrahydrofuran, 15 ml Methanol und 30 ml Wasser werden bei 0 °C 2,53 g (0,06 mol) Lithiumhydroxid-Monohydrat zugegeben. Das Gemisch wird 24 Stunden lang bei 60 °C gerührt. Das Reaktionsgemisch wird anschließend auf eine Phosphatpufferlösung (pH 7,0) gegossen, mit Essigsäureethylester extrahiert und die organische Phase mit einer gesättigten wässrigen Kochsalzlösung gewaschen und sodann über Natriumsulfat getrocknet. Die organische Phase wird bis zur einsetzenden Niederschlagsbildung partiell eingeengt unnd mit Hexan versetzt. Der Niederschlag wird abfiltriert und mit 50 ml Hexan nachgewaschen.
Es werden 5,4 g (89% der Theorie) 3-(5-tert-butoxycarbonylamino-2-ethoxymethoxy-phenyl)-acrylsäure erhalten.
¹H-NMR (300 MHz, DMSO-D₆): δ = 12,4 (br, 1H); 9,22 (br s, 1H); 7,81 (d, 1H); 7,73 (d, 1H); 7,42 (dd, 1H); 7,10 (d, 1H); 6,33 (d, 1H); 5,27 (s, 2H); 3,67 (q, 2H); 1,48 (s, 9H); 1,13 (t, 3H)

### F. Synthese von 3-(5-Amino-2-hydroxyphenyl)-acrylamid Derivaten

Eine Mischung von 0,07g (0,185 mmol) von 3-(5-tert-butoxycarbonylamino-2-ethoxymethoxy-phenyl)-acrylsäure, 0,037g (0,24 mmol) N-Hydroxybenzotriazol-Hydrat und 0,043 g (0,22 mmol) N-(3-Dimethylaminopropyl)-N'-ethyl-carbodiimid-Hydrochlorid wird in Dichlormethan vorgelegt, mit dem entsprechenden Amin (0,22 mmol) sowie mit 0,047 g N-Ethyl-diisopropylamin versetzt und 12 Stunden bei Raumtemperatur geschüttelt. Nach Beendigung der Reaktion wird die Reaktionsmischung in 10 ml Essigsäureethylester gegossen, die organische Phase mit Natriumhydrogencarbonat extrahiert und sodann mit Magnesiumsulfat getrocknet. Das Lösungsmittel wird am Rotationsverdampfer abdestilliert und der Rückstand an Kieselgel mit einem geeigneten Eluiermittel (z.B. Petrolether/Essigsäureethylester oder Dichlormethan/Methanol) gereinigt. Das so erhaltene Produkt wird in 4 ml Ethanol auf 50 °C erwärmt. Anschließend werden zur Herstellung des Hydrochlorides 1,5 ml einer 2,9-molaren ethanolische Salzsäurelösung zugetropft. Die Lösung wird eingeengt und sodann der Rückstand wird getrocknet.

### a. 3-(5-Amino-2-hydroxyphenyl)-N-ethyl-acrylamid-Hydrochlorid

Verwendetes Amin: Ethylamin
Massenspektrum: MH⁺207 (100)

### b. 3-(5-Amino-2-hydroxyphenyl)-N-(4-hydroxyphenyl)-acrylamid-Hydrochlorid

Verwendetes Amin: 4-Hydroxy-benzaldehyd
Massenspektrum: MH⁺271 (100)

### c. 3-(5-Amino-2-hydroxyphenyl)-N-(4-aminopheny)-acrylamid-Hydrochlorid

Verwendetes Amin: 4-Amino-phenyl-carbaminsäure tert-butylester
Massenspektrum: MH⁺270 (100)

### d. N-[4-Amino-2-(2-hydroxyethyl)-2H-pyrazol-3-yl]-3-(5-amino-2-hydroxyphenyl)-acrylamid-Hydrochlorid

Verwendetes Amin: 4,5-Diamino-1-(2-hydroxyethyl)pyrazol
Massenspektrum: MH⁺ 269 (100)

### e. N-Allyl-3-(5-amino-2-hydroxyphenyl)-acrylamid-Hydrochlorid

Verwendetes Amin: Allylamin
Massenspektrum: MH⁺304 (100)

### f. 3-(5-Amino-2-hydroxyphenyl)-N-cyclopropyl-acrylamid-Hydrochlorid

Verwendetes Amin: Cyclopropylamin
Massenspektrum: MH⁺219 (100)

### g. N-(2-Aminoethyl)-3-(5-amino-2-hydroxyphenyl)-acrylamid-Hydrochlorid

Verwendetes Amin: Ethylendiamin
Massenspektrum: MH⁺ 222 (100)

### h. 3-(5-Amino-2-hydroxyphenyl)-N-(4-amino-2(3)-methyl-phenyl)acrylamid-Hydrochlorid

Verwendetes Amin: (4-Amino-2-methylphenyl)-carbaminsäure-tert.butylester und (4-Amino-3-methylphenyl)-carbaminsäure-tert.butylester Massenspektrum: MH⁺284 (100)

### i. 3-(5-Amino-2-hydroxyphenyl)-N-isopropyl-acrylamid-Hydrochlorid

Verwendetes Amin: Isopropylamin
Massenspektrum: MH⁺221 (100)

### j. 3-(5-Amino-2-hydroxyphenyl)-N-propyl-acrylamid Hydrochlorid

Verwendetes Amin: Propylamin
Masspektrum: MH⁺221 (100)

### k. 3-(5-Amino-2-hydroxy-phenyl)-1-pyrrolidin-1-yl-propenon-Hydrochlorid

Verwendetes Amin: Pyrrolidin
Massenspektrum: MH⁺233 (100)

### l. 3-(5-Amino-2-hydroxyphenyl)-N-(2-methoxyethyl)-acrylamid- Hydrochlorid

Verwendetes Amin: 2-Methoxyethylamin
Massenspektrum: MH⁺ 237 (100)

### m. 3-(5-Amino-2-hydroxyphenyl)-1-morpholin-4-yl-propenon-Hydrochlorid

Verwendetes Amin: Morpholin
Massenspektrum: MH⁺249 (100)

### n. 3-(5-Amino-2-hydroxyphenyl)-N-(1-hydroxymethylpropyl)-acrylamid-Hydrochlorid

Verwendetes Amin: 2-Amino-1-butanol
Massenspektrum: MH⁺251 (100)

### o. 3-(5-Amino-2-hydroxy-phenyl)-N-furan-2-yl-methyl-acrylamid-Hydrochlorid

Verwendetes Amin: Furfurylamin
Massenspektrum: MH⁺259 (100)

### p. 3-(5-Amino-2-hydroxyphenyl)-N-methoxy-N-methyl-acrylamid-Hydrochlorid

Verwendetes Amin: N,O-Dimethylhydroxylamin-Hydrochlorid
Massenspektrum: MH⁺223 (100)

### q. 3-(5-Amino-2-hydroxyphenyl)-1-(4-methyl-piperazin-1-yl)-propenon-Hydrochlorid

Verwendetes Amin: 4-Methyl-piperazin
Massenspektrum: MH⁺ 262 (100)

### r. 3-(5-Amino-2-hydroxyphenyl)-1-(4-hydroxy-piperidin-1-yl)-propenon-Hydrochlorid

Verwendetes Amin: 4-Hydroxy-piperidin
Massenspektrum: MH⁺263 (100)

### s. N-(2-Acetylaminoethyl)-3-(5-amino-2-hydroxyphenyl)-acrylamid-Hydrochlorid

Verwendetes Amin: N-Acetylethylendiamin
Massenspektrum: MH⁺264 (100)

### t. 3-(5-Amino-2-hydroxyphenyl)-N-(2-morpholin-4-yl-ethyl)-acrylamid-Hydrochlorid

Verwendetes Amin: 4-(2-Ethylamino)-morpholin
Massenspektrum: MH⁺292 (100)

### u. 3-(5-Amino-2-hydroxyphenyl)-N-[3-(2-oxo-pyrrolidin-1-yl)-propyl]-acrylamid-Hydrochlorid

Verwendetes Amin: 1-(3-aminopropyl)-2-pyrrolidon
Massenspektrum: MH⁺304 (100)

### v. 3-(5-Amino-2-hydroxyphenyl)-N-(2-hydroxy-1-methylethyl)-acrylamid-Hydrochlorid

Verwendetes Amin: 2-Amino-propanol
Massenspektrum: MH⁺237 (100)

### w. 3-(5-Amino-2-hydroxyphenyl)-N-[2-(5-nitro-pyridin-2-ylamino)-ethyl]-acrylamid-Hydrochlorid

Verwendetes Amin: 2-Amino-5-nitro-pyridin
Massenspektrum: MH⁺344 (100)

### x. 3-(5-Amino-2-hydroxy-phenyl)-N-(3-imidazol-1-yl-propyl)-acrylamid Hydrochlorid

Verwendetes Amin: 1-(3-Aminopropyl)-imidazol
Massenspektrum: MH⁺287 (100)

### y. 3-(5-Amino-2-hydroxyphenyl)-N-(tetrahydrofuran-2-yl-methyl)-acrylamid-Hydrochlorid

Verwendetes Amin: Tetrahydrofurfurylamin
Massenspektrum: MH⁺263 (100)

### z. N-[4-Amino-2(3)-(2-hydroxyethyl)-phenyl]-3-(5-amino-2-hydroxyphenyl)-acrylamid-Hydrochlorid

Verwendetes Amin: (4-Amino-2-(2-hydroxyethyl)-phenyl)-carbaminsäuretert.butytester und (4-Amino-3-(2-hydroxyethyl)-phenyl)-carbaminsäuretert.butylester
Massenspektrum: MH⁺314 (100)

### aa. 3-(5-Amino-2-hydroxyphenyl)-N-{4-[bis-(2-hydroxyethyl)-amino]-phenyl}-acrylamid-Hydrochlorid

Verwendetes Amin: 4-Bis-(2-hydroxyethyl)-amino-anilin
Massenspektrum: MH⁺358 (100)

### ab. 3-(5-Amino-2-hydroxyphenyl)-N-(3-aminophenyl)-acrylamid-Hydrochlorid

Verwendetes Amin: (3-Aminophenyl)-carbaminsäure-tert.butylester
Massenspektrum: MH⁺270 (100)

### ac. N-[5-Amino-2(4)-(2-hydroxyethoxy)-phenyl]-3-(5-amino-2-hydroxyphenyl)-acrylamid-Hydrochlorid

Verwendetes Amin: [3-Amino-4-(2-hydroxyethoxy)-phenyl]-carbaminsäure-tert.butylester und [3-Amino-6-(2-hydroxyethoxy)-phenyl]-carbaminsäuretert.butyl-ester
Massenspektrum: MH⁺330 (100)

### ad. 3-(5-Amino-2-hydroxyphenyl)-N-[2-chlor-4-(2-hydroxyethylamino)-5-nitro-phenyl]-acrylamid-Hydrochlorid

Verwendetes Amin: 2-Chlor-4-(2-hydroxyethyl)amino-5-nitro-anilin
Massenspektrum: MH⁺393 (100)

### ae. 3-(5-Amino-2-hydroxyphenyl)-N-benzo[1,3]dioxol-5-yl-acrylamid-Hydrochlorid

Verwendetes Amin: Benzo[1,3]dioxol-5-ylamin
Massenspektrum: MH⁺299 (100)

### af. 3-(5-Amino-2-hydroxyphenyl)-N-(2-hydroxyethyl)-N-methyl-acrylamid-Hydrochlorid

Verwendetes Amin: 2-Methylamino-ethanol
Massenspektrum: MH⁺237 (100)

### ag. 3-(5-Amino-2-hydroxyphenyl)-N-ethyl-N-(2-hydroxyethyl)-acrylamid-Hydrochlorid

Verwendetes Amin: 2-Ethylamino-ethanol
Massenspektrum: MH⁺251 (80)

### ah. 3-(5-Amino-2-hydroxyphenyl)-1-(2-hydroxymethyl-pyrrolidin-1-yl)-propenon-Hydrochlorid

Verwendetes Amin: Prolinol
Massenspektrum: MH⁺263 (100)

### ai. 1-[3-(5-Amino-2-hydroxyphenyl)-acryloyl]-pyrrolidine-2-carbonsäureamid-Hydrochlorid

Verwendetes Amin: Prolinamid
Massenspektrum: MH⁺ 276 (100)

### aj. 3-(5-Amino-2-hydroxyphenyl)-1-(3-hydroxy-piperidin-1-yl)propenon-Hydrochlorid

Verwendetes Amin: 3-Hydroxy-piperidin
Massenspektrum: MH⁺263 (100)

### ak. 3-(5-Amino-2-hydroxyphenyl)-N-(2-hydroxy-1-hydroxymethylethyl)-acrylamid-Hydrochlorid

Verwendetes Amin: 3-Amino-1,2-propandiol
Massenspektrum: MH⁺253 (100)

### al. 2-[3-(5-Amino-2-hydroxyphenyl)-acryloylamino]-3-methyl-buttersäure-Hydrochlorid

Verwendetes Amin: α-Amino-isovaleriansäure
Massenspektrum: MH⁺279 (100)

### am. 3-(5-Amino-2-hydroxyphenyl)-N-(1-carbamoyl-2-hydroxyethyl)-acrylamid-Hydrochlorid

Verwendetes Amin: 2-Amino-3-hydroxy-propionamid
Massenspektrum: MH⁺266 (100)

### an. 3-(5-Amino-2-hydroxyphenyl)-N-(3-hydroxy-4-methyl-phenyl)-acrylamid-Hydrochlorid

Verwendetes Amin: 5-Amino-2-methyl-phenol
Massenspektrum: MH⁺285 (100)

### ao. 3-(5-Amino-2-hydroxyphenyl)-N-(2-hydroxy 5-nitro-phenyl)-acrylamid-Hydrochlorid

Verwendetes Amin: 2-Amino-4-nitro-phenol
Massenspektrum: MH⁺316 (100)

### Beispiele 2 bis 39: Haarfärbemittel

Es werden Haarfärbelösungen der folgenden Zusammensetzung hergestellt:

| | |
|---|---|
| 1,25 mmol | Entwicklersubstanz der Formel (I) gemäß Tabelle 1 |
| 1,25 mmol | Kupplersubstanz gemäß Tabelle 1 |
| 1,0 g | Kaliumoleat (8prozentige wässrige Lösung) |
| 1,0 g | Ammoniak (22prozentige wässrige Lösung) |
| 1,0 g | Ethanol |
| 0, 3 g | Ascorbinsäure |
| ad 100,0 g | Wasser |

30 g der vorstehenden Färbelösung werden unmittelbar vor der Anwendung mit 30 g einer 6prozentigen wässrigen Wasserstoffperoxidlösung vermischt. Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die resultierenden Färbungen sind in Tabelle 1 zusammengefaßt.

**Tabelle 1**

| **Beispiel Nr.** | **Entwickler-substanz der Formel (I)** | **Kupplersubstanz** | | |
|---|---|---|---|---|
| | | II . 1,3-Diamino-4-(2-hydroxy-ethoxy)-benzol-sulfat | III. 5-Amino-2-methyl-phenol | **IV** **. 1-Naphtol** |
| **2.** | Gemäß Beispiel **1a** | rotbraun | rotorange | violett |
| **3.** | gemäß Beispiel **1b** | rotbraun | rotorange | violett |
| **4.** | gemäß Beispiel **1c** | rotbraun | rotorange | violett |
| **5.** | gemäß Beispiel **1d** | rotbraun | rotorange | hellviolett |
| **6.** | gemäß Beispiel **1e** | rotbraun | rotorange | hellviolett |
| **7.** | gemäß Beispiel **1f** | rotbraun | rotorange | hellviolett |
| **8.** | gemäß Beispiel **1g** | rotbraun | rotorange | hellviolett |
| **9.** | gemäß Beispiel **1h** | rotbraun | rotorange | hellviolett |
| **10.** | gemäß Beispiel **1i** | rotbraun | rotorange | hellviolett |
| **11.** | gemäß Beispiel **1j** | rotbraun | rotorange | hellviolett |
| **12.** | gemäß Beispiel **1k** | rotbraun | rotorange | hellviolett |
| **13.** | gemäß Beispiel **1l** | rotbraun | rotorange | hellviolett |
| **14.** | gemäß Beispiel **1m** | rotbraun | rotorange | hellviolett |
| **15.** | gemäß Beispiel **1n** | rotbraun | rotorange | hellviolett |
| **16.** | gemäß Beispiel **1o** | rotbraun | rotorange | hellviolett |
| **17.** | gemäß Beispiel **1p** | rotbraun | rotorange | hellviolett |
| **18.** | gemäß Beispiel **1q** | rotbraun | rotorange | hellviolett |
| **19.** | gemäß Beispiel **1r** | rotbraun | braun | hellviolett |
| **20.** | gemäß Beispiel **1s** | rotbraun | rotbraun | hellviolett |
| **21.** | gemäß Beispiel **1t** | rotbraun | rotorange | hellviolett |
| **22.** | gemäß Beispiel **1u** | rotbraun | rotorange | hellviolett |
| **23.** | gemäß Beispiel **1v** | rotbraun | rotorange | hellviolett |
| **24.** | gemäß Beispiel **1x** | rotbraun | hell-rotorange | hellviolett |
| **25.** | gemäß Beispiel **1y** | rotbraun | hell-rotorange | hellviolett |
| **26.** | gemäß Beispiel **1z** | rotbraun | hell-rotorange | hellviolett |
| **27.** | gemäß Beispiel **1aa** | rotbraun | rot | hellviolett |
| **28.** | gemäß Beispiel **1ab** | rotbraun | hell-rotorange | hellviolett |
| **29.** | gemäß Beispiel **1ac** | rotbraun | hell-rotorange | hellviolett |
| **30.** | gemäß Beispiel **1ae** | rotbraun | hell-rotorange | hellviolett |
| **31.** | gemäß Beispiel **1af** | rotbraun | hell-rotorange | hellviolett |
| **32.** | gemäß Beispiel **1ag** | rotbraun | hell-rotorange | hellviolett |
| **33.** | gemäß Beispiel **1ah** | rotbraun | hell-rotorange | hellviolett |
| **34.** | gemäß Beispiel **1ai** | rotbraun | hell-rotorange | hellviolett |
| **35.** | gemäß Beispiel **1aj** | rotbraun | hell-rotorange | hellviolett |
| **36.** | gemäß Beispiel **1ak** | rotbraun | hell-rotorange | hellviolett |
| **37.** | gemäß Beispiel **1al** | rotbraun | hell-rotorange | hellviolett |
| **38.** | gemäß Beispiel **1am** | rotbraun | hell-rotorange | hellviolett |
| **39.** | gemäß Beispiel **1an** | rotbraun | hell-rotorange | hellviolett |

### Beispiele 40 bis 71: Haarfärbemittel

Es werden Haarfärbelösungen der folgenden Zusammensetzung hergestellt:

| | |
|---|---|
| X g | Entwicklersubstanz **E1** oder **E2** der Formel (I) gemäss Tabelle 3 |
| U g | Entwicklersubstanz **E3** bis **E10** gemäss Tabelle 3 |
| Y g | Kupplersubstanz **K11** bis **K36** gemäss Tabelle 4 |
| Z g | direktziehender Farbstoff **D1** bis **D3** gemäss Tabelle 2 |
| 10,0 g | Kaliumoleat (8prozentige wässrige Lösung) |
| 10,0 g | Ammoniak (22prozentige wässrige Lösung) |
| 10,0 g | Ethanol |
| 0,3 g | Ascorbinsäure |
| ad 100,0 g | Wasser |

30 g der vorstehenden Färbelösung werden unmittelbar vor der Anwendung mit 30 g einer 6prozentigen wässrigen Wasserstoffperoxidlösung vermischt. Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die Färbeergebnisse sind in Tabelle 5 zusammengefasst.

### Beispiele 72 bis 77 Haarfärbemittel

Es werden cremeförmige Farbträgermassen der folgenden Zusammensetzung hergestellt:

| | |
|---|---|
| X g | Entwicklersubstanz **E1** der Formel (I) gemäss Tabelle 3 |
| U g | Entwicklersubstanz **E3** bis **E10** gemäss Tabelle 3 |
| Y g | Kupplersubstanz **K11** bis **K36** gemäss Tabelle 4 |
| Z g | direktziehender Farbstoff **D1** bis **D3** gemäss Tabelle 2 |
| 15,0 g | Cetylalkohol |
| 0,3 g | Ascorbinsäure |
| 3,5 g | Natriumlaurylalkoholdiglycolethersulfat, 28%ige wässrige Lösung |
| 3,0 g | Ammoniak, 22%ige wässrige Lösung |
| 0,3 g | Natriumsulfit, wasserfrei |
| ad 100 g | Wasser |

30 g der vorstehenden Färbecreme werden unmittelbar vor der Anwendung mit 30 g einer 6prozentigen Wasserstoffperoxidlösung vermischt. Anschließend wird das Gemisch auf das Haar aufgetragen. Nach einer Einwirkzeit von 30 Minuten wird das Haar mit Wasser gespült, mit einem handelsüblichen Shampoo gewaschen und getrocknet. Die Färbeergebnisse sind in der nachfolgenden Tabelle 6 zusammengefasst.

**Tabelle 2:**

| **Direkiziehende Farbstoffe** | |
|---|---|
| **D1** | 2,6-Diamino-3-((pyridin-3-yl)azo)pyridin |
| **D2** | 6-Chlor-2-ethylamino-4-nitro-phenol |
| **D3** | 2-Amino-6-chlor-4-nitro-phenol |

**Tabelle 3:**

| **Entwicklersubstanzen** | |
|---|---|
| **E1** | 3-(5-Amino-2-hydroxy-phenyl)-N-ethyl-acrylamid Hydrochlorid |
| **E2** | 3-(5-Amino-2-hydroxy-phenyl)-N-(4-amino-phenyl)-acrylamid Hydrochlorid |
| | |
| **E3** | 2,5-Diamino-phenylethanol-sulfat |
| **E4** | 3-Methyl-4-amino-phenol |
| **E5** | 4-Amino-2-aminomethyl-phenol-dihydrochlorid |
| **E6** | 4-Amino-phenol |
| **E7** | N,N-Bis(2'-hydroxyethyl)-p-phenylendiamin-sulfat |
| **E8** | 4,5-Diamino-1-(2'-hydroxyethyl)-pyrazol-sulfat |
| **E9** | 2,5-Diaminotoluol-sulfat |
| **E10** | 1,4-Diaminobenzol |

**Tabelle 4:**

| **Kupplersubstanzen** | |
|---|---|
| **K11** | 1,3-Diaminobenzol |
| **K12** | 2-Amino-4-(2'-hydroxyethyl)amino-anisol-sulfat |
| **K13** | 1,3-Diamino-4-(2'-Hydroxyethoxy)benzol-sulfat |
| **K14** | 2,4-Diamino-5-fluor-toluol-sulfat |
| **K16** | 3,5-Diamino-2,6-dimethoxy-pyridin-dihydrochlorid |
| **K17** | 2,4-Diamino-5-ethoxy-toluol-sulfat |
| **K18** | N-(3-Dimethylamino)phenylharnstoff |
| **K19** | 1,3-Bis(2,4-Diaminophenoxy)propan-tetrahydrochlorid |
| **K21** | 3-Amino-phenol |
| **K22** | 5-Amino-2-methyl-phenol |
| **K23** | 3-Amino-2-chlor-6-methyl-phenol |
| **K24** | 5-Amino-4-fluor-2-methyl-phenol-sulfat |
| **K25** | 1-Naphthol |
| **K26** | 1-Acetoxy-2-methyl-naphthalin |
| **K31** | 1,3-Dihydroxy-benzol |
| **K32** | 2-Methyl-1,3-dihydroxy-benzol |
| **K33** | 1-Chlor 2,4-dihydroxy-benzol |
| **K34** | 4-(2'-Hydroxyethyl)amino-1,2-methylendioxybenzol-hydrochlorid |
| **K35** | 3,4-Methylendioxy-phenol |
| **K36** | 2-Amino-5-methyl-phenol |

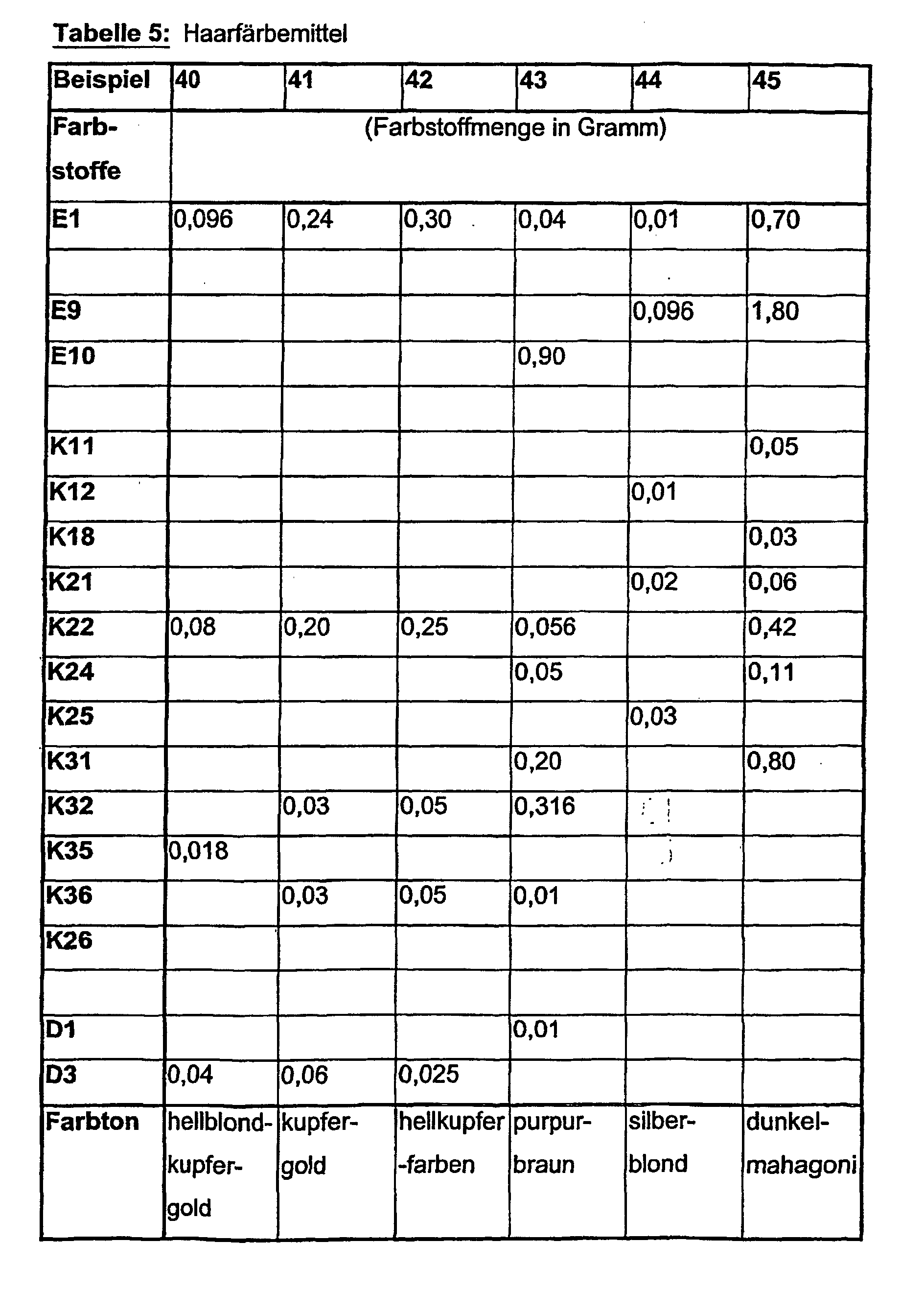

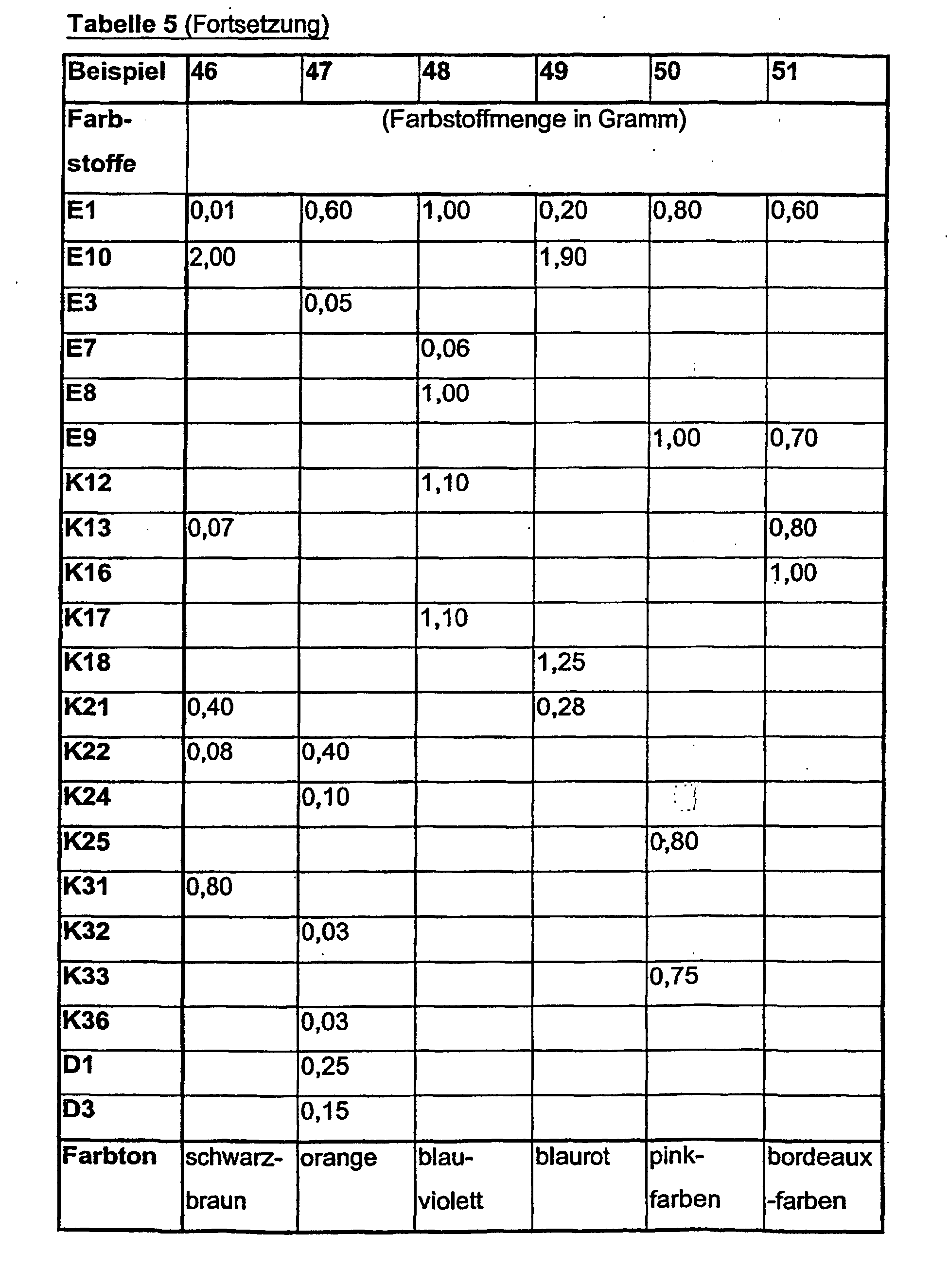

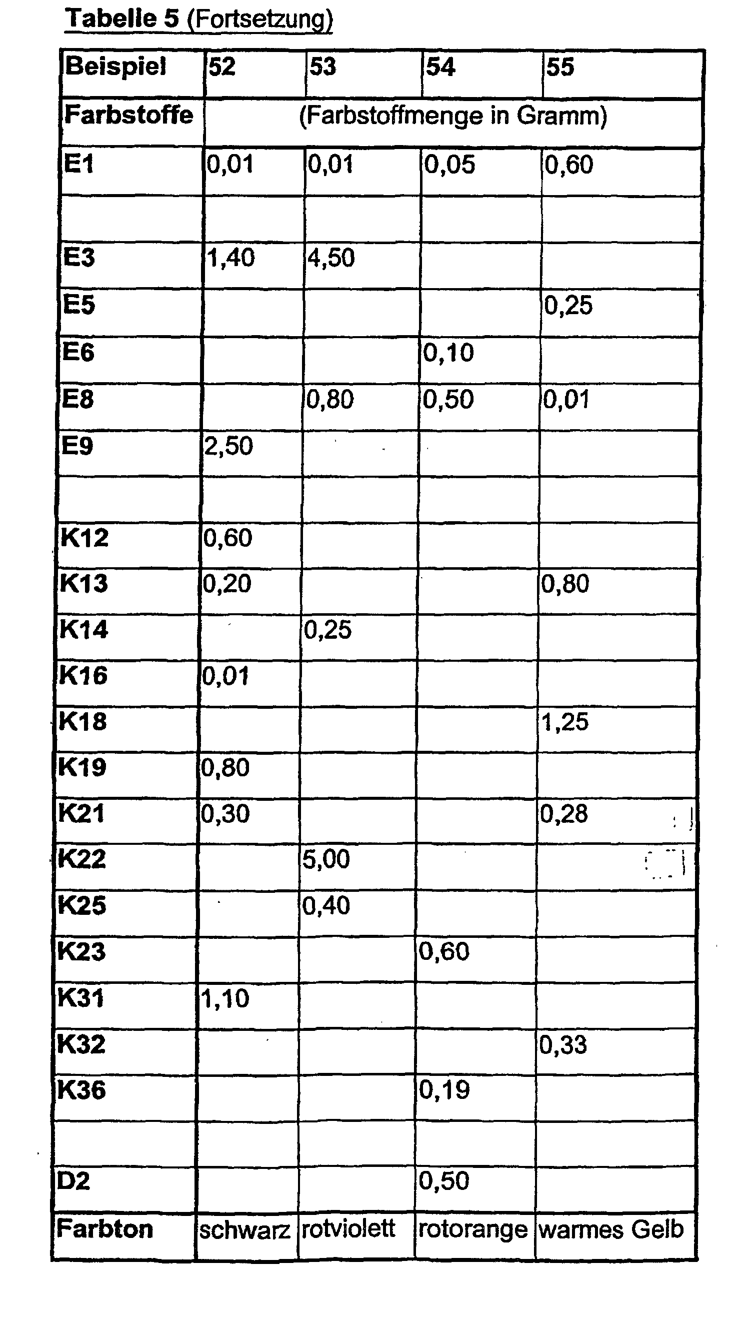

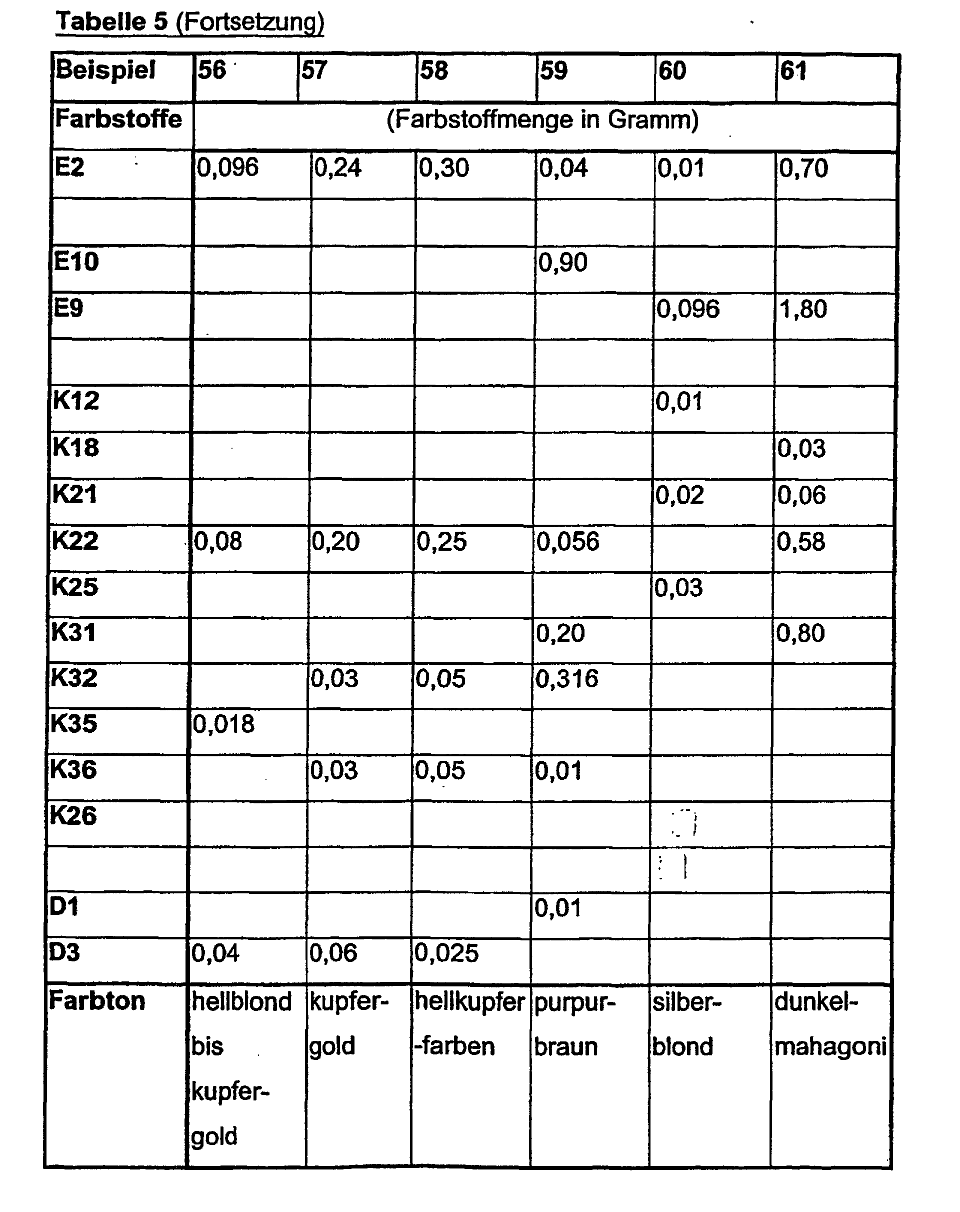

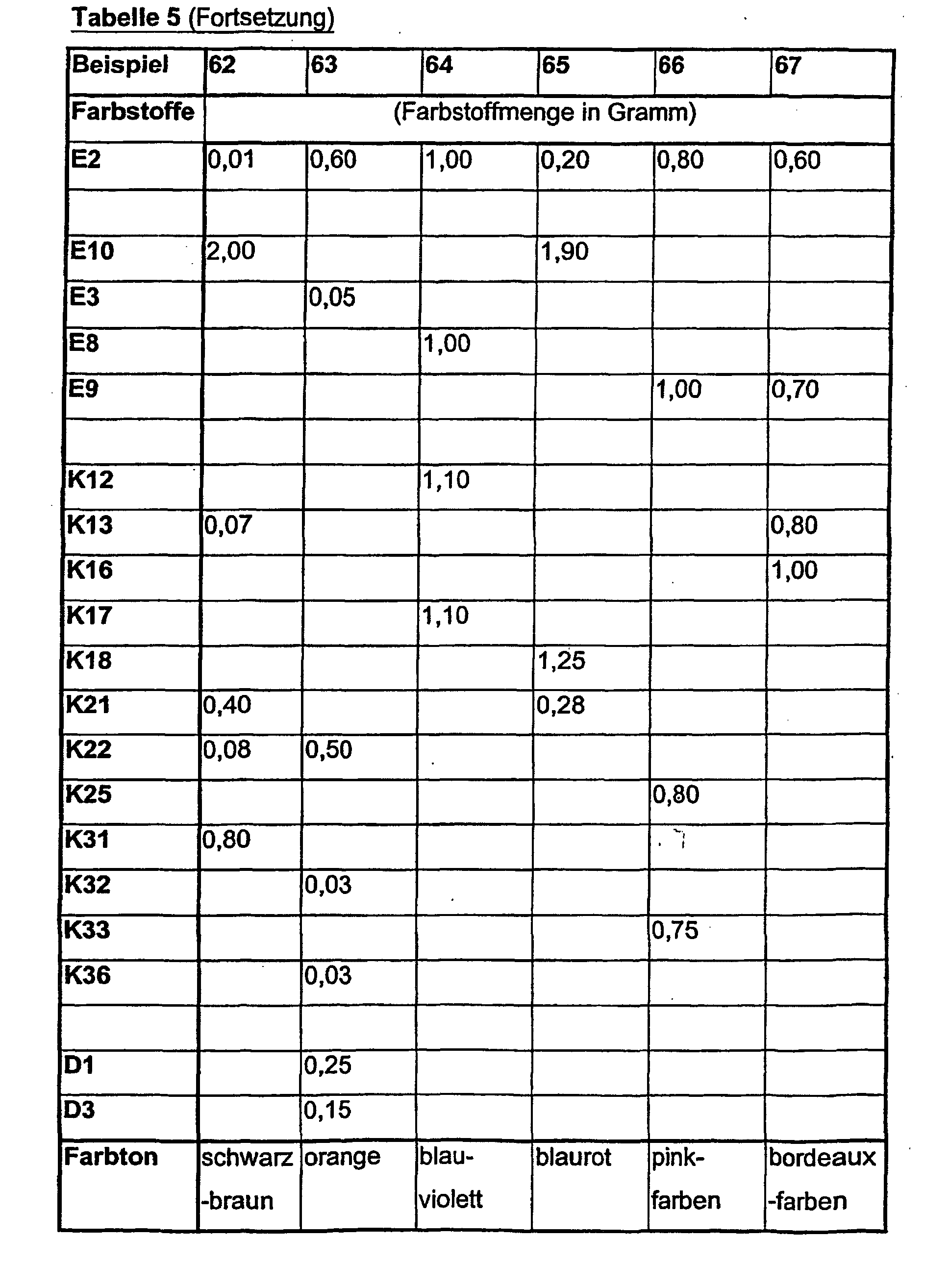

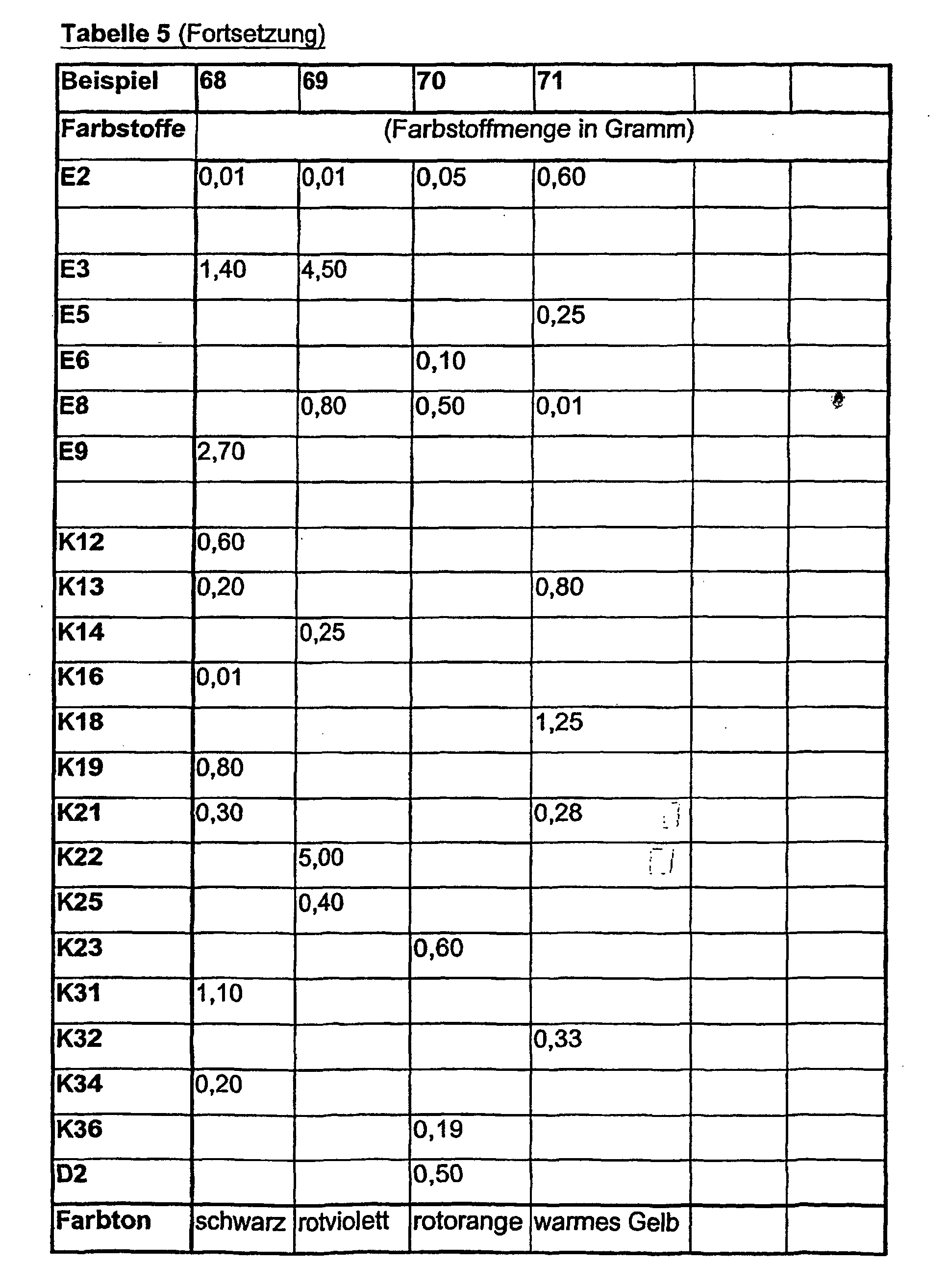

**Tabelle 6:**

| Haarfärbemittel | | | | | | |
|---|---|---|---|---|---|---|
| **Beispiel** | **72** | **73** | **74** | **75** | **76** | **77** |
| **Farbstoffe** | (Farbstoffmenge in Gramm) | | | | | |
| **E1** | 0,10 | 0,20 | 0,01 | 2,00 | 0,50 | 0,70 |
| | | | | | | |
| **E4** | | | | | | 1,60 |
| **E8** | | | | 0,25 | 0,80 | 0,20 |
| **E9** | 3,20 | 1,71 | 0,02 | | | 1,80 |
| | | | | | | |
| **K13** | 0,23 | 0,10 | | | 1,30 | |
| **K14** | 0,20 | | | | | |
| **K16** | | | 0,015 | | | |
| **K21** | 0,40 | 0,80 | | | 0,02 | |
| **K22** | 0,08 | | 0,25 | 1,80 | | 4,50 |
| **K23** | | 0,20 | | | 0,03 | |
| **K31** | 1,05 | 0,135 | 0,02 | 0,25 | | 0,80 |
| **K25** | | | | | | 0,55 |
| **K26** | | | 0,03 | | | |
| **K19** | | | | | 1,70 | |
| **K36** | | 0,27 | | | | |
| | | | | | | |
| **D2** | | 0,01 | | | | |
| **Farbton** | dunkel-braun | schoko-braun | silber-blond | orange-farben | blau-vilett | rotviolett |

Alle in der vorliegenden Anmeldung enthaltenen Prozentangaben stellen soweit nicht anders angegeben Gewichtsprozente dar.

## Patentansprüche

1. (p-Amino-hydroxyphenyl)-acrylamid-Derivate der allgemeinen Formel (I) oder deren physiologisch verträgliche, wasserlösliche Salze, 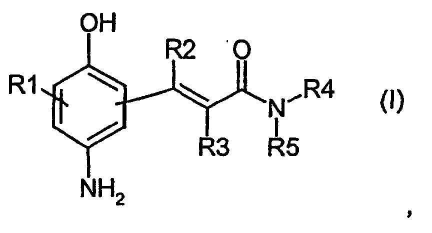 worin
**R1** gleich Wasserstoff, einem Halogenatom, einer C₁-C₄-Alkylgruppe, einer C₁-C₄-Hydroxyalkylgruppe oder einer C₁-C₄-Alkoxygruppe ist;
**R2** und **R3** unabhängig voneinander gleich Wasserstoff oder einer C₁-C₈-Alkylgruppe sind;
**R4** und **R5** unabhänging voneinander gleich Wasserstoff, einer C₁-C₂-Alkoxygruppe, einer C₁-C₆-Alkylgruppe, einer ungesättigten C₃-C₆-Alkylgruppe, einer C₂-C₄-Hydroxyalkylgruppe, einer C₃-C₄-Dihydroxyalkylgruppe, einer C₂-C₄-Aminoalkylgruppe, einer C₂-C₄-Dimethylaminoalkylgruppe, einer C₂-C₄-Acetylaminoalkylgruppe, einer C₂-C₄-Methoxyalkylgruppe, einer C₂-C₄-Ethoxyalkylgruppe, einer C₁-C₄-Cyanoalkylgruppe, einer C₁-C₄-Carboxyalkylgruppe, einer C₁-C₄-Aminocarbonylalkylgruppe, einer Pyridylmethylgruppe, einer Furfurylgruppe, einer Thienylmethylgruppe, einer hydrierten Furfurylgruppe, einer substituierten Pyridylgruppe, einem Rest der Formel (II) 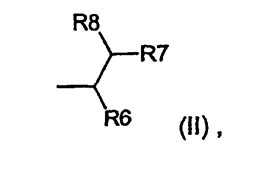 einem Rest der Formel (III) 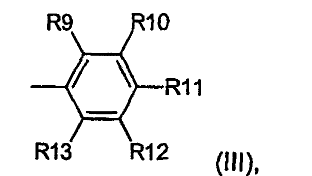 oder einem Rest der Formel (IV) 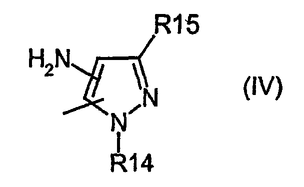 sind, oder R4 und R5 gemeinsam mit dem Stickstoffatom einen Ring der Formel 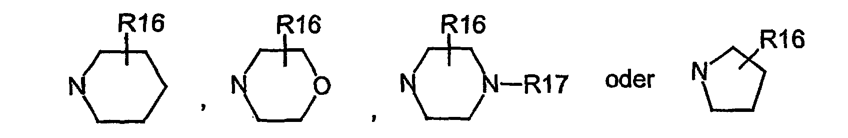 bilden;
**R6** gleich Wasserstoff, einer Carboxygruppe oder einer Aminocarbonylgruppe ist;
**R7** und **R8** unabhänging voneinander gleich Wasserstoff, einer Hydroxygruppe, einer Aminocarbonylgruppe, einer Methylthiomethylgruppe, einem mit einer Phenylgruppe oder einer Hydroxygruppe substituierten Phenylrest oder einem Rest der Formel 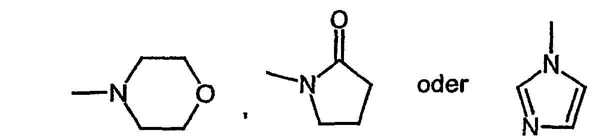 sind
**R9, R10, R11, R12** und **R13** unabhängig voneinander Wasserstoff, ein Halogenatom, eine Cyanogruppe, eine Hydroxygruppe, eine C₁-C₄-Alkoxygruppe, eine C₁-C₄-Hydroxyalkoxygruppe, eine C₁-C₆-Alkylgruppe, eine C₁-C₄ Alkylthioethergruppe, eine Mercaptogruppe, eine Nitrogruppe, eine Aminogruppe, eine Alkylaminogruppe, eine C₁-C₄-Hydroxyalkylaminogruppe, eine Dialkylaminogruppe, eine Di(C₁-C₄-hydroxyalkyl)aminogruppe, eine (C₃ C₄-Dihydroxyalkyl)aminogruppe, eine (C₁-C₄-Hydroxyalkyl)- C₁-C₄-alkylaminogruppe, eine Trifluormethan-gruppe, eine -C(O)H-Gruppe, eine -C(O)CH₃-Gruppe, eine -C(O)CF₃-Gruppe, eine -Si(CH₃)₃-Gruppe, eine C₁-C₄ Hydroxyalkylgruppe oder eine C₃-C₄ Dihydroxyalkylgruppe darstellen, oder zwei nebeneinanderliegende Reste R9 bis R13 eine -O-CH2-O-Brücke bilden;
**R14** gleich einer C₁-C₄-Alkylgruppe einer Benzylgruppe oder einer C₂-C₄ Hydroxyalkylgruppe ist;
**R15** gleich Wasserstoff oder einer C₁-C₆-Alkylgruppe ist;
**R16** gleich Wasserstoff, einer Hydroxygruppe, einer Carboxygruppe, einer Aminocarbonylgruppe oder einer Hydroxymethylgruppe ist; und
**R17** gleich Wasserstoff oder einer C₁-C₆-Alkylgruppe ist;

2. p-Aminophenol-Derivat nach Anspruch 1, **dadurch gekennzeichnet, dass** in der Formel (I) eine, mehrere oder alle Restgruppen **R1, R2** und **R3** gleich Wasserstoff sind.

3. p-Aminophenol-Derivat nach Anspruch 1 oder 2, **dadurch gekennzeichnet, dass** in der Formel (I) R4 gleich einer C₁-C₂-Alkylgruppe, einer Methoxygruppe oder einer C₂-C₄-Hydroxyalkylgruppe ist und R5 gleich einer C₂-C₄-Hydroxyalkylgruppe ist.

4. p-Aminophenol-Derivat nach Anspruch 1 oder 2, **dadurch gekennzeichnet, dass** in der Formel (I) R4 und R5 unabhängig voneinander gleich Wasserstoff, einer C₁-C₄-Alkylgruppe, einer ungesättigen C₁-C₆-Alkylgruppe einer C₂-C₄-Hydroxyalkylgruppe, einer C₃-C₄-Dihydroxyalkylgruppe, einem Furfurylrest, einem substituierten Phenylrest der Formel (III) oder einem substituierten Pyrazolylrest der Formel (IV) sind.

5. p-Aminophenol-Derivat nach Anspruch 1, 2 oder 4, **dadurch gekennzeichnet, dass** in der Formel (I) **R4** gleich Wasserstoff ist und **R5** gleich einer C₁-C₄-Alkylgruppe, einer ungesättigten C₃-C₆-Alkylgruppe, einer C₂-C₄-Hydroxyalkylgruppe, einer C₃-C₄-Dihydroxyalkylgruppe, einem Furfurylrest, einem substituierten Phenylrest der Formel (III) oder einem substituierten Pyrazolylrest der Formel (IV) ist.

6. p-Aminophenol-Derivat nach einem der Ansprüche 1 bis 5, **dadurch gekennzeichnet, dass** es ausgewählt ist aus der Gruppe bestehend aus 3-(5-Amino-2-hydroxyphenyl)-N-ethyl-acrylamid, 3-(2-Amino-5-hydroxy-phenyl)-N-ethyl-acrylamid, 3-(5-Amino-2-hydroxyphenyl)-N-(4-hydroxy-phenyl)-acrylamid, 3-(2-Amino-5-hydroxyphenyl)-N-(4-hydroxyphenyl)-acrylamid, 3-(5-Amino-2-hydroxyphenyl)-N-(4-aminophenyl)-acrylamid, 3-(2-Amino-5-hydroxyphenyl)-N-(4-aminophenyl)-acrylamid, N-[4-Amino-2-(2-hydroxyethyl)-2H-pyrazol-3-yl]-3-(5-amino-2-hydroxyphenyl)-acrylamid und N-[4-Amino-2-(2-hydroxyethyl)-2H-pyrazol-3-yl]-3-(2-amino-5-hydroxyphenyl)-acrylamid sowie deren physiologisch verträglichen Salzen.

7. Mittel zum oxidativen Färben von Keratinfasem auf der Basis einer Entwicklersubstanz-Kupplersubstanz-Kombination, **dadurch gekennzeichnet, dass** es als Entwicklersubstanz mindestens ein p-Aminophenol-Derivat der Formel (I) nach einem der Ansprüche 1 bis 6 enthält.

8. Mittel nach Anspruch 7, **dadurch gekennzeichnet, dass** es das p-Aminophenol-Derivat der Formel (I) in einer Menge von 0,005 bis 20 Gewichtsprozent enthält.

9. Mittel nach Anspruch 7 oder 8, **dadurch gekennzeichnet, dass** die Kupplersubstanz ausgewählt ist aus der Gruppe bestehend aus N-(3-Dimethylamino-phenyl)-hamstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methylbenzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 1,3-Diamino-4-(3-hydroxypropoxy)-benzol, 1,3-Diamino-4-(2-methoxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methylphenol, 5-Amino-4-methoxy-2-methyl-phenol, 5-Amino-4-ethoxy-2-methylphenol, 3-Amino-2,4-dichlor-phenol, 5-Amino-2,4-dichlor-phenol, 3-Amino-2-methyl-phenol, 3-Amino-2-chlor 6-methyl-phenol, 3-Amino-phenol, 2-[(3-Hydroxyphenyl)amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)-aminol-2-methyl-phenol, 2-Amino-3-hydroxy-pyridin, 2,6-Dihydroxy-3,4-dimethylpyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphtholacetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxaz)n, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion.

10. Mittel nach einem der Ansprüche 7 bis 9, **dadurch gekennzeichnet, dass** es zusätzlich zu den Verbindungen der Formel (I) noch weitere bekannte enthält, welche ausgewählt sind aus der Gruppe bestehend aus 1,4-Diamino-benzol, 1,4-Diamino-2-methyl-benzol, 1,4-Diamino-2,6-dimethyl-benzol, 1,4-Diamino-3,5-diethyl-benzol, 1,4-Diamino-2,5-dimethyl-benzol, 1,4-Diamino-2,3-dimethyl-benzol, 2-Chlor-1,4-diaminobenzol, 1,4-Diamino-2-(thiophen-2-yl)benzol, 1,4-Diamino-2-(thiophen-3-yl)benzol, 1,4-Diamino-2-(pyridin-3-yl)benzol, 2,5-Diaminobiphenyl, 1,4-Diamino-2-methoxymethyl-benzol, 1,4-Diamino-2-aminomethyl-benzol, 1,4-Diamino-2-hydroxymethyl-benzol, 1,4-Diamino-2-(2-hydroxyethoxy)-benzol, 2-(2-(Acetylamino)ethoxy)-1,4-diamino-benzol, 4-Phenylamino-anilin, 4-Dimethylamino-anilin, 4-Diethylamino-anilin, 4-Dipropylamino-anilin, 4-[Ethyl(2-hydroxyethyl)-amino]-anilin, 4-[Di(2-hydroxyethyl)amino]-anilin, 4-[Di(2-hydroxyethyl)-amino]-2-methylanilin, 4-[(2-Methoxyethyl)amino]-anilin, 4-[(3-Hydroxy-propyl)amino]-anilin, 4-[(2,3-Dihydroxypropyl)amino]-anilin, 1,4-Diamino-2-(1-hydroxyethyl)-benzol, 1,4-Diamino-2-(2-hydroxyethyl)-benzol, 1,4-Diamino-2-(1-methylethyl)-benzol, 1,3-Bis[(4-aminophenyl)(2-hydroxyethyl)amino]-2-propanol, 1,4-Bis[(4-Aminophenyl)amino]-butan, 1,8-Bis(2,5-diaminophenoxy)-3,6-dioxaoctan, 4-Amino-phenol, 4-Amino-3-methyl-phenol, 4-Amino-3-(hydroxymethyl)-phenol, 4-Amino-3-fluor-phenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxyethyl)-amino]methyl-phenol, 4-Amino-2-methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1H-pyrazol, 4,5-Diamino-1-(1-methylethyl)-1H-pyrazol, 4,5-Diamino-1-[(4-methylphenyl)methyl]-1Hpyrazol, 1-[(4-Chlorphenyl)methyl]-4,5-diamino-1H-pyrazol, 4,5-Diamino-1-methyl-1H-pyrazol, 2-Amino-phenol, 2-Amino-6-methyl-phenol, 2-Amino-5-methyl-phenol und 1,2,4-Trihydroxy-benzol.

11. Mittel nach einem der Ansprüche 7 bis 10, **dadurch gekennzeichnet, dass** die Entwicklersubstanzen und Kupplersubstanzen, bezogen auf die Gesamtmenge des Färbemittels, jeweils in einer Gesamtmenge von 0,005 bis 20 Gewichtsprozent enthalten sind.

12. Mittel nach einem der Ansprüche 7 bis 11, **dadurch gekennzeichnet, dass** es zusätzlich mindestens einen direktziehenden Farbstoff enthält.

13. Mittel nach einem der Ansprüche 7 bis 12, **dadurch gekennzeichnet, dass** es ein Haarfärbemittel ist.

## Claims

1. (p-Aminohydroxyphenyl)acrylamide derivatives of the general formula (I) or physiologically compatible, water-soluble salts thereof, 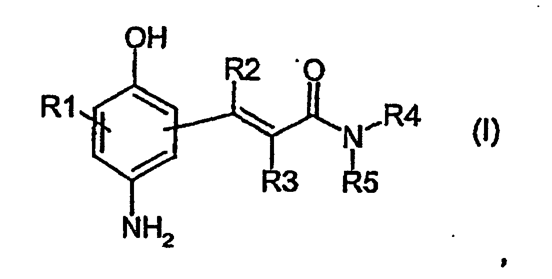 in which
**R1** is hydrogen, a halogen atom, a C₁-C₄-alkyl group, a C₁-C₄-hydroxyalkyl group or a C₁-C₄-alkoxy group;
**R2** and **R3**, independently of one another, are hydrogen or a C₁-C₆-alkyl group;
**R4** and **R5,** independently of one another, are hydrogen, a C₁-C₂-alkoxy group, a C₁-C₆-alkyl group, an unsaturated C₃-C₆-alkyl group, a C₂-C₄-hydroxyalkyl group, a C₃-C₄-dihydroxyalkyl group, a C₂-C₄-aminoalkyl group, a C₂-C₄-dimethylaminoalkyl group, a C₂-C₄-acetylaminoalkyl group, a C₂-C₄-methoxyalkyl group, a C₂-C₄-ethoxyalkyl group, a C₁-C₄-cyanoalkyl group, a C₁-C₄-carboxyalkyl group, a C₁-C₄-aminocarbonylalkyl group, a pyridylmethyl group, a furfuryl group, a thienylmethyl group, a hydrogenated furfuryl group, a substituted pyridyl group, a radical of the formula (II) 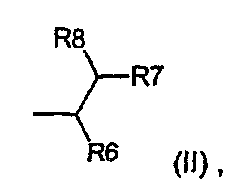 a radical of the formula (III) 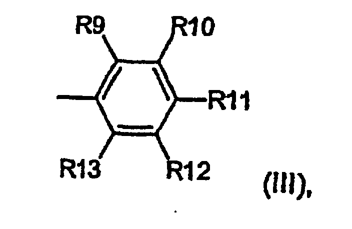 or a radical of the formula (IV) 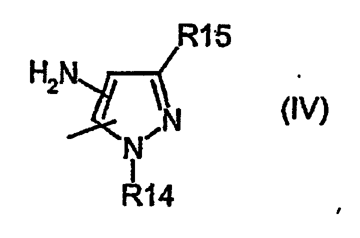 or R4 and R5 together with the nitrogen atom form a ring of the formula 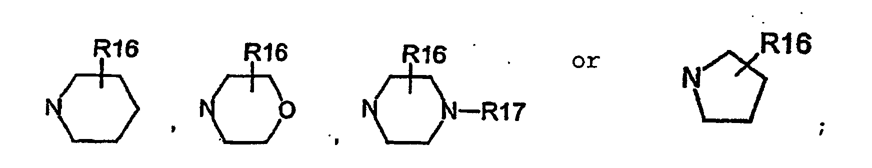 **R6** is hydrogen, a carboxy group or an aminocarbonyl group;
**R7** and **R8**, independently of one another, are hydrogen, a hydroxy group, an aminocarbonyl group, a methylthiomethyl group, a phenyl radical substituted by a phenyl group or a hydroxy group, or a radical of the formula 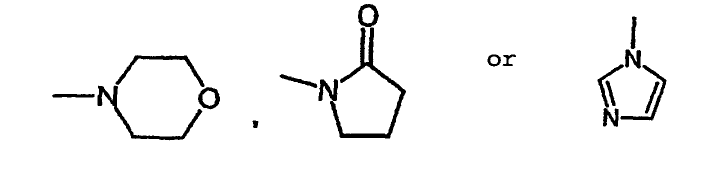 **R9, R10, R11, R12** and **R13,** independently of one another, are hydrogen, a halogen atom, a cyano group, a hydroxy group, a C₁-C₄-alkoxy group, a C₁-C₄-hydroxy-alkoxy group, a C₁-C₆-alkyl group, a C₁-C₄-alkylthioether group, a mercapto group, a nitro group, an amino group, an alkylamino group, a C₁-C₄-hydroxyalkylamino group, a dialkylamino group, a di(C₁-C₄-hydroxyalkyl)amino group, a (C₃-C₄-dihydroxyalkyl) amino group, a (C₁-C₄-hydroxy-alkyl)-C₁-C₄-alkylamino group, a trifluoromethane group, a -C(O)H group, a -(C(O)CH₃ group, a -C(O)CF₃ group, a Si(CH₃)₃ group, a C₁-C₄-hydroxyalkyl group or a C₃-C₄ dihydroxyalkyl group, or two adjacent radicals R9 to R13 form an -O-CH₂-O bridge;
**R14** is a C₁-C₄-alkyl group, a benzyl group or a C₂-C₄-hydroxyalkyl group;
**R15** is hydrogen or a C₁-C₆-alkyl group;
**R16** is hydrogen, a hydroxy group, a carboxy group, an aminocarbonyl group or a hydroxymethyl group; and
**R17** is hydrogen or a C₁-C₆-alkyl group.

2. p-Aminophenol derivative according to Claim 1, **characterized in that**, in the formula (I), one, more or all of the radical groups **R1, R2** and **R3** are hydrogen.

3. p-Aminophenol derivative according to Claim 1 or 2, **characterized in that**, in the formula (I), **R4** is a C₁-C₂-alkyl group, a methoxy group or a C₂-C₄-hydroxy-alkyl group, and **R5** is a C₂-C₄-hydroxyalkyl group.

4. p-Aminophenol derivative according to Claim 1 or 2, **characterized in that**, in the formula (I) **R4** and **R5,** independently of one another, are hydrogen, a C₁-C₄-alkyl group, an unsaturated C₁-C₆-alkyl group, a C₂-C₄-hydroxyalkyl group, a C₃-C₄-dihydroxyalkyl group, a furfuryl radical, a substituted phenyl radical of the formula (III) or a substituted pyrazolyl radical of the formula (IV).

5. p-Aminophenol derivative according to Claim 1, 2 or 4, **characterized in that**, in the formula (I), **R4** is hydrogen and **R5** is a C₁-C₄-alkyl group, an unsaturated C₃-C₆-alkyl group, a C₂-C₄-hydroxyalkyl group, a C₃-C₄-dihydroxyalkyl group, a furfuryl radical, a substituted phenyl radical of the formula (III) or a substituted pyrazolyl radical of the formula (IV).

6. p-Aminophenol derivative according to one of Claims 1 to 5, **characterized in that** it is chosen from the group consisting of 3-(5-amino-2-hydroxyphenyl)-N-ethylacrylamide, 3-(2-amino-5-hydroxyphenyl)-N-ethyl-acrylamide, 3-(5-amino-2-hydroxyphenyl)-N-(4-hydroxy-phenyl)acrylamide, 3-(2-amino-5-hydroxyphenyl)-N-(4-hydroxyphenyl)acrylamide, 3-(5-amino-2-hydroxyphenyl)-N-(4-aminophenyl)acrylamide, 3-(2-amino-5-hydroxy-phenyl)-N-(4-aminophenyl)acrylamide, N-[4-amino-2-(2-hydroxyethyl)-2H-pyrazol-3-yl]-3-(5-amino-2-hydroxy-phenyl)acrylamide and N-[4-amino-2-(2-hydroxyethyl)-2H-pyrazol-3-yl]-3-(2-amino-5-hydroxyphenyl)acrylamide and physiologically compatible salts thereof.

7. Agent for the oxidative colouring of keratin fibres based on a developer substance-coupler substance combination, **characterized in that** it comprises, as developer substance, at least one p-aminophenol derivative of the formula (I) according to one of Claims 1 to 6.

8. Agent according to Claim 7, **characterized in that** it comprises the p-aminophenol derivative of the formula (I) in an amount of from 0.005 to 20 percent by weight.

9. Agent according to Claim 7 or 8, **characterized in that** the coupler substance is chosen from the group consisting of N-(3-dimethylaminophenyl)urea, 2,6-diaminopyridine, 2-amino-4-[(2-hydroxyethyl)amino]-anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxy-ethoxy)-5-methylbenzene, 2,4-di[(2-hydroxyethyl)amino]-1,5-dimethoxybenzene, 2,3-diamino-6-methoxypyridine, 3-amino-6-methoxy-2-(methylamino)pyridine, 2,6-diamino-3,5-dimethoxypyridine, 3,5-diamino-2,6-dimethoxy-pyridine, 1,3-diaminobenzene, 2,4-diamino-1-(2-hydroxy-ethoxy)benzene, 1,3-diamino-4-(2,3-dihydroxypropoxy)-benzene, 1,3-diamino-4-(3-hydroxypropoxy)benzene, 1,3-diamino-4-(2-methoxyethoxy)benzene, 2,4-diamino-1,5-di(2-hydroxyethoxy)benzene, 1-(2-aminoethoxy)-2,4-diaminobenzene, 2-amino-1-(2-hydroxyethoxy)-4-methylaminobenzene, 2,4-diaminophenoxyacetic acid, 3-[di(2-hydroxyethyl)amino]aniline, 4-amino-2-di[(2-hydroxyethyl)amino]-1-ethoxybenzene, 5-methyl-2-(1-methylethyl)phenol, 3-[(2-hydroxyethyl)amino]-aniline, 3-[(2-aminoethyl)amino]aniline, 1,3-di(2,4-diaminophenoxy)propane, -di(2,4-diaminophenoxy)methane, 1,3-diamino-2,4-dimethoxybenzene, 2,6-bis(2-hydroxy-ethyl)aminotoluene, 4-hydroxyindole, 3-dimethylamino-phenol, 3-diethylaminophenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methylphenol, 3-amino-2-chloro-6-methylphenol, 3-aminophenol, 2-[(3-hydroxyphenyl)amino]acetamide, 5-[(2-hydroxyethyl)amino]-4-methoxy-2-methylphenol, 5-[(2-hydroxyethyl)amino]-2-methylphenol, 3-[(2-hydroxyethyl)amino]phenol, 3-[(2-methoxyethyl)-amino]phenol, 5-amino-2-ethylphenol, 5-amino-2-methoxyphenol, 2-(4-amino-2-hydroxyphenoxy)ethanol, 5-[(3-hydroxypropyl)amino]-2-methylphenol, 3-[(2,3-dihydroxypropyl)amino]-2-methylphenol, 3-[(2-hydroxyethyl)amino]-2-methylphenol, 2-amino-3-hydroxy-pyridine, 2,6-dihydroxy-3,4-dimethylpyridine, 5-amino-4-chloro-2-methylphenol, 1-naphthol, 2-methyl-1-naphthol, 1,5-dihydroxynaphthalene, 1,7-dihydroxynaphthalene, 2,3-dihydroxynaphthalene, 2,7-dihydroxynaphthalene, 2-methyl-1-naphthol acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 3,4-methylenedioxyphenol, 3,4-methylenedioxyaniline, 5-[(2-hydroxyethyl)amino]-1,3-benzodioxol, 6-bromo-1-hydroxy-3,4-methylenedioxy-benzene, 3,4-diaminobenzoic acid, 3,4-dihydro-6-hydroxy-1,4(2H)-benzoxazine, 6-amino-3,4-dihydro-1,4(2H)benzoxazine, 3-methyl-1-phenyl-5-pyrazolone, 5,6-dihydroxyindole, 5,6-dihydroxyindoline, 5-hydroxyindole, 6-hydroxyindole, 7-hydroxyindole and 2,3-indolenedione.

10. Agent according to one of claims 7 to 9, **characterized in that**, in addition to the compounds of the formula (I), it also comprises further known compounds which are chosen from the group consisting of 1,4-diaminobenzene, 1,4-diamino-2-methylbenzene, 1,4-diamino-2,6-dimethylbenzene, 1,4-diamino-3,5-diethylbenzene, 1,4-diamino-2,5-dimethylbenzene, 1,4-diamino-2,3-dimethylbenzene, 2-chloro-1,4-diaminobenzene, 1,4-diamino-2-(thiophen-2-yl)benzene, 1,4-diamino-2-(thiophen-3-yl)benzene, 1,4-diamino-2-(pyridin-3-yl)benzene, 2,5-diaminobiphenyl, 1,4-diamino-2-methoxymethylbenzene, 1,4-diamino-2-aminomethylbenzene, 1,4-diamino-2-hydroxymethylbenzene, 1,4-diamino-2-(2-hydroxyethoxy)benzene, 2-(2-(acetylamino)ethoxy)-1,4-diaminobenzene, 4-phenylaminoaniline, 4-dimethylamino-aniline, 4-diethylaminoaniline, 4-dipropylaminoaniline, 4-[ethyl(2-hydroxyethyl)amino]aniline, 4-[di(2-hydroxy-ethyl)amino]aniline, 4-[di(2-hydroxyethyl)amino]-2-methylaniline, 4-[(2-methoxyethyl)amino]aniline, 4-[(3-hydroxypropyl)amino]aniline, 4-[(2,3-dihydroxypropyl)-amino]aniline, 1,4-diamino-2-(1-hydroxyethyl)benzene, 1,4-diamino-2-(2-hydroxyethyl)benzene, 1,4-diamino-2-(1-methylethyl)benzene, 1,3-bis[(4-aminophenyl)-(2-hydroxyethyl)amino]-2-propanol, 1,4-bis[(4-amino-phenyl)amino]butane, 1,8-bis(2,5-diaminophenoxy)-3,6-dioxaoctane, 4-aminophenol, 4-amino-3-methylphenol, 4-amino-3-(hydroxymethyl)phenol, 4-amino-3-fluoro-phenol, 4-methylaminophenol, 4-amino-2-(aminomethyl)-phenol, 4-amino-2-(hydroxymethyl)phenol, 4-amino-2-fluorophenol, 4-amino-2-[(2-hydroxyethyl)amino]-methylphenol, 4-amino-2-methylphenol, 4-amino-2-(methoxymethyl)phenol, 4-amino-2-(2-hydroxyethyl)-phenol, 5-aminosalicylic acid, 2,5-diaminopyridine, 2,4,5,6-tetraaminopyrimidine, 2,5,6-triamino-4-(1H)-pyrimidone, 4,5-diamino-1-(2-hydroxyethyl)-1H-pyrazol, 4,5-diamino-1-(1-methylethyl)-1H-pyrazol, 4,5,-diamino-1-[(4-methylphenyl)methyl]-1H-pyrazol, 1-[(4-chloro-phenyl)methyl]-4,5-diamino-1H-pyrazol, 4,5-diamino-1-methyl-1H-pyrazol, 2-aminophenol, 2-amino-6-methyl-phenol, 2-amino-5-methylphenol and 1,2,4-trihydroxy-benzene.

11. Agent according to one of Claims 7 to 10, **characterized in that** the developer substances and the coupler substances, based on the total amount of the colorant, are in each case present in a total amount of from 0.005 to 20 percent by weight.

12. Agent according to one of Claims 7 to 11, **characterized in that** it additionally comprises at least one direct dye.

13. Agent according to one of Claims 7 to 12, **characterized in that** it is a hair colorant.

## Revendications

1. Dérivés de (p-aminohydroxyphényl)acrylamide de formule générale (I) ou leurs sels physiologiquement acceptables, solubles dans l'eau, 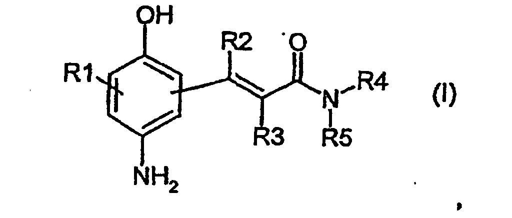 dans laquelle
R1 représente hydrogène, un atome d'halogène, un groupement alkyle en C₁ à C₄, un groupement hydroxyalkyle en C₁ à C₄ ou un groupement alcoxy en C₁ à C₄ ;
R2 et R3 représentent indépendamment l'un de l'autre hydrogène ou un groupement alkyle en C₁ à C₆ ;
R4 et R5 représentent indépendamment l'un de l'autre hydrogène, un groupement alcoxy en C₁ à C₂, un groupement alkyle en C₁ à C₆, un groupement alkyle en C₃ à C₆ insaturé, un groupement hydroxyalkyle en C₂ à C₄, un groupement dihydroxyalkyle en C₃ à C₄, un groupe aminoalkyle en C₂ à C₄, un groupement diméthylaminoalkyle en C₂ à C₄, un groupement acétylaminoalkyle en C₂ à C₄, un groupement méthoxyalkyle en C₂ à C₄, un groupement éthoxyalkyle en C₂ à C₄, un groupement cyanoalkyle en C₁ à C₄, un groupement carboxyalkyle en C₁ à C₄, un groupement aminocarbonylalkyle en C₁ à C₄, un groupement pyridylméthyle, un groupement furfuryle, un groupement thiénylméthyle, un groupement furfuryle hydrogéné, un groupement pyridyle substitué, un radical de formule (II) 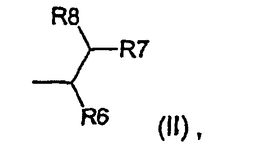 un radical de formule (III) 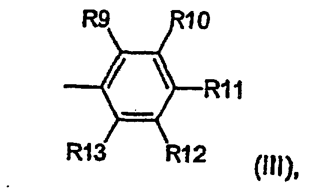 ou un radical de formule (IV) 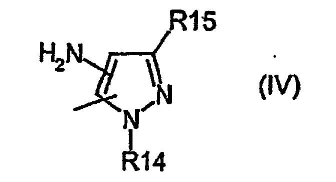 ou R4 et R5 forment ensemble avec l'atome d'azote un cycle de formule 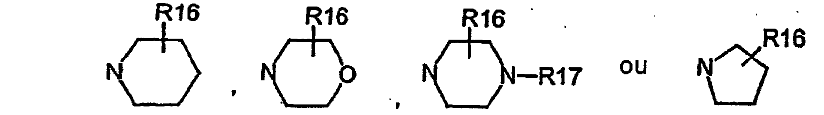 R6 représente hydrogène, un groupement carboxy ou un groupement aminocarbonyle ;
R7 et R8 représentent indépendamment l'un de l'autre hydrogène, un groupement hydroxy, un groupement aminocarbonyle, un groupement méthylthiométhyle, un radical phényle substitué par un groupement phényle ou un Groupement hydroxy ou un radical de formule 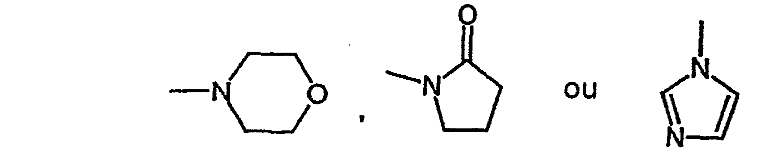 R9, R10, R11, R12 et R13 représentent indépendamment l'un de l'autre hydrogène, un atome d'halogène, un groupement cyano, un groupement hydroxy, un groupement alcoxy en C₁ à C₄, un groupement hydroxyalcoxy en C₁ à C₄, un groupement alkyle en C₁ à C₆, un groupement (alkyle en C₁ à C₄)thioéther, un groupement mercapto, un groupement nitro, un groupement amino, un groupement alkylamino, un groupement hydroxy(alkyle en C₁ à C₄)amino, un groupement dialkylamino, un groupement di(hydroxyalkyle en C₁ à C₄) amino, un groupement (dihydroxyalkyle en C₃ à C₄)amino, un groupement (hydroxyalkyle en C₁ à C₄) (alkyle en C₁ à C₄) amino, un groupement trifluorométhane, un groupement -C(O)H, un groupement -C(O)CH₃, un groupement -C(O)CF₃, un groupement -Si(CH₃)₃, un groupement hydroxyalkyle en C₁ à C₄ ou un groupement dihydroxyalkyle en C₃ à C₄ ou deux radicaux adjacents R9 à R13 forment un pont -O-CH₂-O- ;
R14 représente un groupement alkyle en C₁ à C₄, un groupement benzyle ou un groupement hydroxyalkyle en C₂ à C_{4 ;}
R15 représente hydrogène ou un groupement alkyle en C₁ à C_{6 ;}
R16 représente hydrogène, un groupement hydroxy, un groupement carboxy, un groupement aminocarbonyle ou un groupement hydroxyméthyle ; et
R17 représente hydrogène ou un groupement alkyle en C₁ à C₆.

2. Dérivé de p-aminophénol selon la revendication 1, **caractérisé en ce que** dans la formule (I), un, plusieurs ou tous les radicaux R1, R2 et R3 représentent hydrogène.

3. Dérivé de p-aminophénol selon la revendication 1 ou 2, **caractérisé en ce que** dans la formule (I), R4 représente un groupement alkyle en C₁ à C₂, un groupement méthoxy ou un groupement hydroxyalkyle en C₂ à C₄, et R5 représente un groupement hydroxyalkyle en C₂ à C₄.

4. Dérivé de p-aminophénol selon la revendication 1 ou 2, **caractérisé en ce que** dans la formule (I), R4 et R5 représentent indépendamment l'un de l'autre hydrogène, un groupement alkyle en C₁ à C₄, un groupement alkyle en C₁ à C₆ insaturé, un groupement hydroxyalkyle en C₂ à C₄, un groupement dihydroxyalkyle en C₃ à C₄, un radical furfuryle, un radical phényle substitué de formule (III) ou un radical pyrazolyle substitué de formule (IV).

5. Dérivé de p-aminophénol selon la revendication 1, 2 ou 4, **caractérisé en ce que** dans la formule (I), R4 représente hydrogène et R5 représente un groupement alkyle en C₁ à C₄, un groupement alkyle en C₃ à C₆ insaturé, un groupement hydroxyalkyle en C₂ à C₄, un groupement dihydroxyalkyle en C₃ à C₄, un radical furfuryle, un radical phényle substitué de formule (III) ou un radical pyrazolyle substitué de formule (IV).

6. Dérivé de p-aminophénol selon l'une quelconque des revendications 1 à 5, **caractérisé en ce qu'**il est choisi dans le groupe constitué par le 3-(5-amino-2-hydroxyphényl)-N-éthylacrylamide, le 3-(2-amino-5-hydroxyphényl)-N-éthylacrylamide, le 3-(5-amino-2-hydroxyphényl)-N-(4-hydroxyphényl)acrylamide, le 3-(2-amino-5-hydroxyphényl)-N-(4-hydroxyphényl)acrylamide, le 3-(5-amino-2-hydroxyphényl)-N-(4-aminophényl)-acrylamide, le 3-(2-amino-5-hydroxyphényl)-N-(4-amino-phényl)acrylamide, le N-[4-amino-2-(2-hydroxyéthyl)-2H-pyrazol-3-yl]-3-(5-amino-2-hydroxyphényl)acrylamide et le N-[4-amino-2-(2-hydroxyéthyl)-2H-pyrazol-3-yl]-3-(2-amino-5-hydroxyphényl)acrylamide ainsi que leurs sels physiologiquement acceptables.

7. Agent pour la teinture par oxydation de fibres kératiniques à base d'une combinaison de substance de développement et de substance de couplage, **caractérisé en ce qu'**il contient comme substance de développement au moins un dérivé de p-aminophénol de formule (I) selon l'une quelconque des revendications 1 à 6.

8. Agent selon la revendication 7, **caractérisé en ce qu'**il contient le dérivé de p-aminophénol de formule (I) en une quantité de 0,005 à 20% en poids.

9. Agent selon la revendication 7 ou 8, **caractérisé en ce que** la substance de couplage est choisie dans le groupe constitué par la N-(3-diméthyl-aminophényl)urée, la 2,6-diaminopyridine, le 2-amino-4-[(2-hydroxyéthyl)amino]anisole, le 2,4-diamino-1-fluoro-5-méthylbenzène, le 2,4-diamino-1-méthoxy-5-méthylbenzène, le 2,4-diamino-1-éthoxy-5-méthylbenzène, le 2,4-diamino-1-(2-hydroxyéthoxy)-5-méthylbenzène, le 2,4-di[(2-hydroxyéthyl)amino]-1,5-diméthoxybenzène, la 2,3-diamino-6-méthoxypyridine, la 3-amino-6-méthoxy-2-(méthylamino)pyridine, la 2,6-diamino-3,5-diméthoxy-pyridine, la 3,5-diamino-2,6-diméthoxypyridine, le 1,3-diaminobenzène, le 2,4-diamino-1-(2-hydroxyéthoxy)-benzène, le 1,3-diamino-4-(2,3-dihydroxypropoxy)-benzène, le 1,3-diamino-4-(3-hydroxypropoxy)benzène, le 1,3-diamino-4-(2-méthoxyéthoxy)benzène, le 2,4-diamino-1,5-di(2-hydroxyéthoxy)benzène, le 1-(2-aminoéthoxy)-2,4-diaminobenzène, le 2-amino-1-(2-hydroxyéthoxy)-4-méthylaminobenzène, l'acide 2,4-diaminophénoxyacétique, la 3-[di(2-hydroxyéthyl)amino]aniline, le 4-amino-2-di[(2-hydroxyéthyl)amino]-1-éthoxybenzène, le 5-méthyl-2-(1-méthyléthyl)phénol, la 3-[(2-hydroxyéthyl)amino]-aniline, la 3-[(2-aminoéthyl)aminolaniline, le 1,3-di(2,4-diaminophénoxy)propane, le di(2,4-diamino-phénoxy)méthane, le 1,3-diamino-2,4-diméthoxy-benzène, le 2,6-bis(2-hydroxyéthyl)aminotoluène, le 4-hydroxyindole, le 3-diméthylaminophénol, le 3-diéthylaminophénol, le 5-amino-2-méthylphénol, le 5-amino-4-fluoro-2-méthylphénol, le 5-amino-4-méthoxy-2-méthylphénol, le 5-amino-4-éthoxy-2-méthylphénol, le 3-amino-2,4-dichlorophénol, le 5-amino-2,4-dichlorophénol, le 3-amino-2-méthylphénol, le 3-amino-2-chloro-6-méthylphénol, le 3-aminophénol, le 2-[(3-hydroxyphényl)amino]acétamide, le 5-[(2-hydroxyéthyl)amino]-4-méthoxy-2-méthylphénol, le 5-[(2-hydroxyéthyl)amino]-2-méthylphénol, le 3-[(2-hydroxyéthyl)amino]phénol, le 3-[(2-méthoxyéthyl)amino]phénol, le 5-amino-2-éthylphénol, le 5-amino-2-méthoxyphénol, le 2-(4-amino-2-hydroxyphénoxy)éthanol, le 5-[(3-hydroxypropyl)amino]-2-méthylphénol, le 3-[(2,3-di-hydroxypropyl)amino]-2-méthylphénol, le 3-[(2-hydroxy-éthyl)amino]-2-méthylphénol, la 2-amino-3-hydroxy-pyridine, la 2,6-dihydroxy-3,4-diméthylpyridine, le 5-amino-4-chloro-2-méthylphénol, le 1-naphtol, le 2-méthyl-1-naphtol, le 1,5-dihydroxynaphtalène, le 1,7-dihydroxynaphtalène, le 2,3-dihydroxynaphtalène, le 2,7-dihydroxynaphtalène, le 1-naphtolacétate de 2-méthyle, le 1,3-dihydroxybenzène, le 1-chloro-2,4-dihydroxybenzène, le 2-chloro-1,3-dihydroxybenzène, le 1,2-dichloro-3,5-dihydroxy-4-méthylbenzène, le 1,5-dichloro-2,4-dihydroxybenzène, le 1,3-dihydroxy-2-méthylbenzène, le 3,4-méthylènedioxyphénol, la 3,4-méthylènedioxyaniline, le 5-[(2-hydroxyéthyl)amino]-1,3-benzodioxole, le 6-bromo-1-hydroxy-3,4-méthylène-dioxybenzène, l'acide 3,4-diaminobenzoïque, la 3,4-dihydro-6-hydroxy-1,4(2H)-benzoxazine, la 6-amino-3,4-dihydro-1,4(2H)-benzoxazine, la 3-méthyl-1-phényl-5-pyrazolone, le 5,6-dihydroxyindole, la 5,6-dihydroxyindoline, le 5-hydroxyindole, le 6-hydroxyindole, le 7-hydroxyindole et la 2,3-indolinedione.

10. Agent selon l'une quelconque des revendications 7 à 9, **caractérisé en ce qu'**il contient, en plus des composés de formule (I) encore d'autres composés connus, qui sont choisis dans le groupe constitué par le 1,4-diaminobenzène, le 1,4-diamino-2-méthylbenzène, le 1,4-diamino-2,6-diméthylbenzène, le 1,4-diamino-3,5-diéthylbenzène, le 1,4-diamino-2,5-diméthylbenzène, le 1,4-diamino-2,3-diméthylbenzène, le 2-chloro-1,4-diaminobenzène, le 1,4-diamino-2-(thiophén-2-yl)benzène, le 1,4-diamino-2-(thiophén-3-yl)benzène, le 1,4-diamino-2-(pyridin-3-yl)benzène, le 2,5-diamino-diphényle, le 1,4-diamino-2-méthoxyméthylbenzène, le 1,4-diamino-2-aminométhylbenzène, le 1,4-diamino-2-hydroxyméthylbenzène, le 1,4-diamino-2-(2-hydroxyéthoxy)benzène, le 2-(2-(acétylamino)éthoxy)-1,4-diaminobenzène, la 4-phénylaminoaniline, la 4-diméthylaminoaniline, la 4-diéthylaminoaniline, la 4-dipropylaminoaniline, la 4-[éthyl(2-hydroxyéthyl)-amino]aniline, la 4-[di(2-hydroxyéthyl)amino]aniline, la 4-[di(2-hydroxyéthyl)amino]-2-méthylaniline, la 4-[(2-méthoxyéthyl)amino]aniline, la 4-[(3-hydroxy-propyl)amino]aniline, la 4-[(2,3-dihydroxypropyl)-amino]aniline, le 1,4-diamino-2-(1-hydroxyéthyl)-benzène, le 1,4-diamino-2-(2-hydroxyéthyl)benzène, le 1,4-diamino-2-(1-méthyléthyl)benzène, le 1,3-bis[(4-aminophényl)(2-hydroxyéthyl)amino]-2-propanol, le 1,4-bis[(4-aminophényl)amino]butane, le 1,8-bis(2,5-diaminophénoxy)-3,6-dioxaoctane, le 4-aminophénol, le 4-amino-3-méthylphénol, le 4-amino-3-(hydroxyméthyl)-phénol, le 4-amino-3-fluorophénol, le 4-méthylamino-phénol, le 4-amino-2-(aminométhyl)phénol, le 4-amino-2-(hydroxyméthyl)phénol, le 4-amino-2-fluorophénol, le 4-amino-2-[(2-hydroxyéthyl)amino]méthylphénol, le 4-amino-2-méthylphénol, le 4-amino-2-(méthoxyméthyl)-phénol, le 4-amino-2-(2-hydroxyéthyl)phénol, l'acide 5-aminosalicylique, la 2,5-diaminopyridine, la 2,4,5,6-tétraaminopyrimidine, le 2,5,6-triamino-4-(1H)-pyrimidone, le 4,5-diamino-1-(2-hydroxyéthyl)-1H-pyrazole, le 4,5-diamino-1-(1-méthyléthyl)-1H-pyrazole, le 4,5-diamino-1-[(4-méthylphényl)méthyl)-1H-pyrazole, le 1-[(4-chlorophényl)méthyl]-4,5-diamino-1H-pyrazole, le 4,5-diamino-1-méthyl-1H-pyrazole, le 2-aminophénol, le 2-amino-6-méthylphénol, le 2-amino-5-méthylphénol et le 1,2,4-trihydroxybenzène.

11. Agent selon l'une quelconque des revendications 7 à 10, **caractérisé en ce que** les substances de développement et les substances de couplage, par rapport à la quantité totale de la teinture, sont à chaque fois contenues en une quantité totale de 0,005 à 20% en poids.

12. Agent selon l'une quelconque des revendications 7 à 11, **caractérisé en ce qu'**il contient en plus au moins un colorant direct.

13. Agent selon l'une quelconque des revendications 7 à 12, **caractérisé en ce qu'**il s'agit d'une teinture pour les cheveux.
